(19) Europäisches Patentamt
European Patent Office
Office européen des brevets

(11) **EP 3 640 864 A1**

(12) **EUROPEAN PATENT APPLICATION**

(43) Date of publication:
**22.04.2020 Bulletin 2020/17**

(51) Int Cl.:
**G06N 5/02** (2006.01)     **G06N 5/00** (2006.01)
**G16B 40/30** (2019.01)

(21) Application number: **18201367.2**

(22) Date of filing: **18.10.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **FUJITSU LIMITED**
**Kanagawa 211-8588 (JP)**

(72) Inventors:
• **MITSUISHI, Yutaka**
**Kawasaki-shi, Kanagawa 211-8588 (JP)**

• **NOVÁCEK, Vit**
**Co. Galway (IE)**
• **VANDENBUSSCHE, Pierre-Yves**
**Co. Galway (IE)**

(74) Representative: **Haseltine Lake Kempner LLP**
**Lincoln House, 5th Floor**
**300 High Holborn**
**London WC1V 7JH (GB)**

Remarks:
Amended claims in accordance with Rule 137(2) EPC.

(54) **A COMPUTER-IMPLEMENTED METHOD AND APPARATUS FOR INFERRING A PROPERTY OF A BIOMEDICAL ENTITY**

(57)    A computer-implemented method of inferring a property of a biomedical entity comprising: inputting two or more knowledge graphs related to biomedical entities in the form of vertices and edges and having different arities, at least one of the knowledge graphs being directional and at least one of the knowledge graphs being labelled; converting each of the knowledge graphs to a hyperedge representation as a list of unique edge identifiers, each with any source and target vertex vertices, any vertices connected by the edge in an undirected manner, and any labels associated with the edge; converting the hyperedge representations into a bipartite graph with the vertices and labels in one set and the edges in the other set, wherein the vertices and labels are linked to the edges as per the hyperedge representations by auxiliary edges A; sampling the bipartite graph to embed the bipartite graph in a feature matrix of the vertices, labels and edges; using the feature matrix to infer a property of a biomedical entity in the combined knowledge graphs based on the matrix entries.

Figure 1

**Description**

The technical field of the present invention is the analysis of knowledge graphs (graph-based means for large-scale robust representation of noisy, incomplete knowledge). A particular application is the explanation of biological or biomedical entities such as protein and protein interactions, especially human and animal proteins. Increasingly large amounts of human knowledge are being represented as machine-readable graph data sets. Graph representation allows for convenient and robust modelling of real world knowledge, but also brings specific computational challenges, such as the need to cope with the relational and multivariate nature of the data representation. One of the most promising recent approaches is to compute knowledge graph embeddings - low-rank continuous vector representations of the knowledge graph entities and/or relations that may then be used as a proxy for efficient implementation of a broad range of knowledge discovery techniques. Embeddings may be produced by graph traversal and may facilitate efficient computation in tasks like knowledge graph completion or discovery of patterns (such as clusters or outliers).

The specific ways for computing embeddings for knowledge graphs have only started to be investigated quite recently, and there is still much space for improvement. There are several challenges in the emergent field of knowledge graph embeddings. Recently, knowledge graphs with relations/relationships (edges in the graph) that may involve more than two nodes (also referred to herein as vertices) and mixed directionality of the relation/link between them (some directional and some non-directional relationships) between these nodes have become available. The prior art embedding techniques cannot effectively manage such graphs.

In the bio-informatics domain, this problem is particularly present. For example, along with databases representing protein interactions (undirected binary relations connecting two nodes only), sit some more complex pathway databases with indication of protein interactions in the context of a pathway (ternary relationship involving two proteins and a pathway labelling their relation as a context argument). Similarly other databases such as gene-disease (directed binary relations connecting a gene to a disease) are juxtaposed with co-occurrence relations with provenance extracted from biomedical literature (ternary undirected relationship involving two genes or a gene and disease in the context of a scientific publication). Computing knowledge graph embeddings from this heterogeneous (mixed arity and mixed directionality) information is a main challenge.

According to an embodiment of a first aspect of the invention, there is provided a computer-implemented method of inferring a property of a biomedical entity comprising: inputting two or more knowledge graphs related to biomedical entities in the form of vertices and edges and having different arities, at least one of the knowledge graphs being directional and at least one of the knowledge graphs being labelled; converting each of the knowledge graphs to a hyperedge representation as a list of unique edge identifiers, each with any source and target vertex vertices, any vertices connected by the edge in an undirected manner, and any labels associated with the edge; converting the hyperedge representations into a bipartite graph with the vertices and labels in one set and the edges in the other set, wherein the vertices and labels are linked to the edges as per the hyperedge representations by auxiliary edges A; sampling the bipartite graph to embed the bipartite graph as matrix entries in a feature matrix of the vertices, labels and edges; and using the feature matrix to infer a property of a biomedical entity in the combined knowledge graphs based on the matrix entries.

The term "biomedical entity" is used herein to cover physical biomedical entities and their states, and modulators. They may be divided into subgroups such as biological molecules, cells, viruses, organisms, medical conditions, pharmaceutical etc.

With regard to molecules, embodiments may relate, for example, to mutated DNA molecule" as a physical product or to "a mutation" as an abstract concept or a property of the DNA molecule.

Biological molecules may include, for example:

- nucleic acids (including DNA, RNA)
- amino acids
- polypeptides
- proteins (made of 1 or more polypeptides, which are chains of amino acids)

Organisms may include:

- bacteria
- plants
- animals

Medical conditions may include:

- diseases
- physical injuries

- physical conditions (such as pregnancy)

Pharmaceuticals may include any type of chemical drug or biotherapeutic agent.

The biomedical entity may alternatively be viewed as anything related to a biomedical process.

The biomedical entity may be a protein. Especially proteins and in particular human proteins appear in many different knowledge graphs. They may be vertices and/or edges in some or all of the graphs used in invention embodiments. Combining different graphs according to the embodiments allows the production of embeddings which provide extra information about protein interactions which may be used to infer protein properties (such as interrelationships or presence in the same biological pathway, such as metabolic, gene expression or signalling pathway).

One knowledge graph input may be, for example, a directed labelled graph with binary-only relationships, such as protein interaction data and another knowledge graph input may be a mixed directed/undirected labelled graph with ternary relationships such as a pathway database.

Any suitable embedding method may be used. Hence, a graph sampler is to sample the graph (using any known method) to allow the production of embeddings in the form of a matrix of vertices, labels and edges, and the embeddings are used to infer a property of a biomedical entity such as a protein.

In one invention embodiment sampling the bipartite graph includes: traversing the bipartite graph to provide possible sequences along the links between the sets. Embedding the bipartite graph may include: producing a feature matrix of the vertices, labels and edges from the sequences, with a co-occurrence score forming a matrix entry in each cell of the feature matrix. In this case, the feature matrix may be used to infer a property of a biomedical entity in the knowledge graph based on the co-occurrence scores.

The hyperedge representations (of the two or more input graphs) may be combined before conversion into a bipartite graph, by considering vertices having the same name as the same vertex. If there are alternative names for the same vertex they may be grouped using a mapping tool.

Each edge identifier may have the form

$$(e, S, T, U)^l$$

where $e$ is a unique edge identifier, $S, T$ are sets of source and target vertices, respectively, that are connected by the edge in a directed manner, $U$ is a set of vertices that are connected by the edge in an undirected manner, and $l$ is an edge label.

An auxiliary edge $A$ may be formed between each vertex and an edge to which it is joined (in the original input graphs), with a relation between two vertices including two auxiliary edges, and the edge. That is, the auxiliary edges (which may be directed or undirected, according to whether the edge is directed or not) are between the first vertex and the edge and the edge and the second vertex.

An auxiliary edge may be formed between a label for an edge and the edge itself. In this case, it need not be directed.

Traversal of the bipartite graph may produce sequences which alternate between a vertex or edge label and an edge of the hyperedge representations (travelling along the auxiliary edges, which do not however appear in the sequences). Thus the edges, edge labels and vertices of the original graphs become entities (vertices) in the bipartite graph. The sequences may be of any suitable maximum length.

The feature matrix is populated, for example, by creating co-occurrence scores taking into account the distance in a sequence between two entities in the sequence and adding co-occurrence scores into the feature matrix for each sequence.

This procedure may use a context window, starting at each point in a sequence but the last to give context sequences; with the co-occurrence scores between each starting point and the subsequent entities in the context sequence being calculated taking into account the distance between the entities in the context sequence and added into the feature matrix. Thus the matrix may be build up cumulatively, sequence by sequence.

The last step is inferring information from the feature matrix. The property of the protein being considered may be inferred by calculation of a similarity of two or more proteins, for example using cosine similarity (of the rows of the feature matrix for the two proteins)

According to an embodiment of a second aspect of the invention, there is provided an apparatus to infer a property of a biomedical entity comprising: memory and a processor providing a bipartite converter, a graph sampler and a distributional analyser; wherein: the bipartite converter is to: receive two or more knowledge graphs related to biomedical entities in the form of vertices and edges and having different arities, at least one of the knowledge graphs being directional and at least one of the knowledge graphs being labelled; convert each of the knowledge graphs to a hyperedge representation as a list of unique edge identifiers, each with any source and target vertex vertices, any vertices connected by the edge in an undirected manner, and any labels associated with the edge; and to convert the hyperedge representations into a bipartite graph with the vertices and labels in one set and the edges in the other set, wherein the vertices and labels are linked to the edges as per the hyperedge representations by auxiliary edges $A$; the graph sampler is to sample the bipartite graph; and the distributional analyser is to: embed the bipartite graph as matrix entries in a feature matrix of the vertices, labels and edges; to store the feature matrix in the memory; and to use the feature matrix to infer a property of a biomedical entity in the combined knowledge graphs based on the matrix entries.

In many embodiments, the biomedical entity is a protein and the related knowledge graphs are all directly or indirectly related to proteins.

The reader will appreciate that the graph techniques described above may equally be applied to other information and are not restricted to biomedical entities and information. The input graphs may represent any form of information. Properties of anything represented by vertices or edges of the original graph may be inferred. In the general sense, items and the relations between them may be represented in the graphs and these items and relations may be of any type: biomedical, chemical, physical, conceptual, mathematical, financial etc.

Thus according to an aspect of a more general embodiment, there is provided a computer-implemented method of inferring a property of an item comprising: inputting two or more knowledge graphs related to items in the form of vertices and edges and having different arities, at least one of the knowledge graphs being directional and at least one of the knowledge graphs being labelled; converting each of the knowledge graphs to a hyperedge representation as a list of unique edge identifiers, each with any source and target vertex vertices, any vertices connected by the edge in an undirected manner, and any labels associated with the edge; converting the hyperedge representations into a bipartite graph with the vertices and labels in one set and the edges in the other set, wherein the vertices and labels are linked to the edges as per the hyperedge representations by auxiliary edges A; sampling the bipartite graph to embed the bipartite graph as matrix entries in a feature matrix of the vertices, labels and edges; and using the feature matrix to infer a property of an item in the combined knowledge graphs based on the matrix entries.

Furthermore, according to an aspect of a further more general embodiment, there is provided an apparatus to infer a property of an item comprising: memory and a processor providing a bipartite converter, a graph sampler and a distributional analyser; wherein: the bipartite converter is to: receive two or more knowledge graphs related to items in the form of vertices and edges and having different arities, at least one of the knowledge graphs being directional and at least one of the knowledge graphs being labelled; convert each of the knowledge graphs to a hyperedge representation as a list of unique edge identifiers, each with any source and target vertex vertices, any vertices connected by the edge in an undirected manner, and any labels associated with the edge; and to convert the hyperedge representations into a bipartite graph with the vertices and labels in one set and the edges in the other set, wherein the vertices and labels are linked to the edges as per the hyperedge representations by auxiliary edges $A$; the graph sampler is to sample the bipartite graph; and the distributional analyser is to: embed the bipartite graph as matrix entries in a feature matrix of the vertices, labels and edges; to store the feature matrix in the memory; and to use the feature matrix to infer a property of an item in the combined knowledge graphs based on the matrix entries.

Sub-aspects of the more general embodiments correspond to sub-aspects of the biomedical embodiments described above.

Also, a single-graph embodiment of the invention relates to a situation in which a single graph may be input and processed. Such an embodiment retains the advantage of the graph processing including the hyperedge representation and reformulation to a bipartite graph without necessarily inputting more than one graph. The graph may relate to biomedical entities such as proteins, or to other items.

Thus according to an aspect of this single-graph embodiment, there is provided a computer-implemented method of inferring a property of an item comprising: inputting one or more knowledge graphs related to items in the form of vertices and edges; converting the one or more knowledge graph to one or more hyperedge representations as a list of unique edge identifiers, each with any source and target vertex vertices, any vertices connected by the edge in an undirected manner, and any labels associated with the edge; converting the one or more hyperedge representations into a bipartite graph with the vertices and labels in one set and the edges in the other set, wherein the vertices and labels are linked to the edges as per the one or more hyperedge representations by auxiliary edges A; sampling the bipartite graph to embed the bipartite graph as matrix entries in a feature matrix of the vertices, labels and edges; and using the feature matrix to infer a property of an item in the one or more knowledge graphs based on the matrix entries.

Furthermore, according to an aspect of a further single-graph embodiment, there is provided an apparatus to infer a property of an item comprising: memory and a processor providing a bipartite converter, a graph sampler and a distributional analyser; wherein: the bipartite converter is to: receive one or more knowledge graphs related to items in the form of vertices and edges; convert the one or more knowledge graphs to one or more hyperedge representation as a list of unique edge identifiers, each with any source and target vertex vertices, any vertices connected by the edge in an undirected manner, and any labels associated with the edge; and to convert the one or more hyperedge representation into a bipartite graph with the vertices and labels in one set and the edges in the other set, wherein the vertices and labels are linked to the edges as per the hyperedge representations by auxiliary edges $A$; the graph sampler is to sample the bipartite graph; and the distributional analyser is to: embed the bipartite graph as matrix entries in a feature matrix of the vertices, labels and edges; to store the feature matrix in the memory; and to use the feature matrix to infer a property of a biomedical entity in the one or more knowledge graphs based on the matrix entries.

An apparatus or computer program according to preferred embodiments of the present invention may comprise any combination of the method aspects. Equally, any of the methods set out above may be combined. Methods or computer programs according to further embodiments may be described as computer-implemented in that they require processing and memory capability.

The apparatus according to preferred embodiments is described as configured or arranged to, or simply "to" carry out certain functions. This configuration or arrangement could be by use of hardware or middleware or any other suitable system. In preferred embodiments, the configuration or arrangement is by software. The apparatus may be divided into a bipartite converter, a graph sampler and a distributional analyser to distinguish between the different functions carried out in processing of the graphs.

Thus according to one aspect there is provided a program which, when loaded onto at least one computer configures the computer to become the apparatus according to any of the preceding apparatus definitions or any combination thereof.

According to a further aspect there is provided a program which when loaded onto the at least one computer configures the at least one computer to carry out the method steps according to any of the preceding method definitions or any combination thereof.

In general the computer may comprise the elements listed as being configured or arranged to provide the functions defined. For example this computer may include memory, processing, and optionally a network interface to receive the knowledge graphs.

The invention may be implemented in digital electronic circuitry, or in computer hardware, firmware, software, or in combinations of them. The invention may be implemented as a computer program or computer program product, i.e., a computer program tangibly embodied in a non-transitory information carrier, e.g., in a machine-readable storage device, or in a propagated signal, for execution by, or to control the operation of, one or more hardware modules.

A computer program may be in the form of a stand-alone program, a computer program portion or more than one computer program and may be written in any form of programming language, including compiled or interpreted languages, and it may be deployed in any form, including as a stand-alone program or as a module, component, subroutine, or other unit suitable for use in a data processing environment. A computer program may be deployed to be executed on one module or on multiple modules at one site or distributed across multiple sites and interconnected by a communication network.

Method steps of the invention may be performed by one or more programmable processors executing a computer program to perform functions of the invention by operating on input data and generating output. Apparatus of the invention may be implemented as programmed hardware or as special purpose logic circuitry, including e.g., an FPGA (field programmable gate array) or an ASIC (application-specific integrated circuit).

Processors suitable for the execution of a computer program include, by way of example, both general and special purpose microprocessors, and any one or more processors of any kind of digital computer. Generally, a processor will receive instructions and data from a read-only memory or a random access memory or both. The essential elements of a computer are a processor for executing instructions coupled to one or more memory devices for storing instructions and data.

The invention is described in terms of particular embodiments. Other embodiments are within the scope of the following claims. For example, the steps of the invention may be performed in a different order and still achieve desirable results.

Elements of the invention have been described using the terms "processor", "memory", etc. The skilled person will appreciate that such functional terms and their equivalents may refer to parts of the system that are spatially separate but combine to serve the function defined. Equally, the same physical parts of the system may provide two or more of the functions defined. For example, separately defined functions may be implemented using the same memory and/or processor as appropriate.

Preferred features of the present invention will now be described, purely by way of example, with references to the accompanying drawings, in which:-

Figure 1 is an overview flow chart of an invention embodiment;
Figure 2 is a block diagram of an invention embodiment;
Figure 3 is an overview of the method with inputs and some outputs;
Figure 4 is an example of graph structure representations;
Figure 5 is a view a standard graph and multigraph for merging;
Figure 6 is the result of merging the graphs of Figure 5;
Figure 7 is a standard graph with the path *Pg* (direct and bipartite representation);
Figure 8 is a hypergraph with the path *Ph* (bipartite representation);

Figure 9 is an overview flow chart of a specific embodiment;
Figure 10 is a standard graph in a graphical and a list form;
Figure 11 is a hypergraph in a graphical and a list form;
Figure 12 is a flow chart of conversion to a bipartite graph;
Figure 13 is a hypergraph in a graphical and a list form;
Figure 14 is the bipartite version of the Figure 13 hypergraph, in a graphical and a list form;
Figure 15 is a flow chart of graph sampling;
Figure 16 is a representation of all the sequences generated from the graph of Figures 13 and 14;
Figure 17 is the hypergraph of Figure 13 without edge label *Co* in a graphical and a list form;
Figure 18 is the bipartite version of the Figure 17 hypergraph, in a graphical and a list form;
Figure 19 is a representation of all the sequences generated from the graph of Figures 16 and 17;
Figure 20 is the hypergraph of Figure 13 without the directions, in a graphical and a list form;
Figure 21 is the bipartite version of the Figure 20 hypergraph, in a graphical and a list form;
Figure 22 is a representation of some sequences generated from the graph of Figures 20 and 21;
Figure 23 is a representation of further sequences generated from the graph of Figures 20 and 21;
Figure 24 is a representation of the remaining sequences generated from the graph of Figures 20 and 21;
Figure 25 is a flow chart of distributional analysis;
Figure 26 is a zeroed feature matrix;
Figure 27 is a feature matrix after a first sequence is embedded according to invention embodiments;
Figure 28 is the feature matrix after a second sequence is embedded;
Figure 29 is the feature matrix after a third sequence is embedded;
Figure 30 is a completed feature matrix;
Figure 31 is a feature matrix from a second example corresponding to prior art;
Figure 32 is a feature matrix from a third example corresponding to prior art; and
Figure 33 is a block diagram of an apparatus for use with invention embodiments.

Recently, knowledge graphs with relationships that may involve more than two nodes and mixed directionality between these nodes have become available (examples are pathway knowledge graphs or co-occurrence networks with provenance). In the prior art there is a lack of robust methods for processing these knowledge graphs with statements of arbitrary arity and directionality.

There are no known methods for producing embeddings for such structures. An ideal embedding method should distinguish between directed and undirected relationships and support both directed and undirected connections within one hyperedge. This is especially important for modelling of context-dependent directed binary relationships in the above-mentioned datasets where the context argument (such as a pathway in which nodes can be connected to other nodes primarily connected by a relation) does not typically have any meaningful directionality with respect to the main arguments (the nodes).

The prior art techniques cannot effectively address graphs of mixed arity and directionality. Embodiments present a solution to this problem: a technique for uniform transformation of knowledge graphs of varying levels of complexity into structures (i.e. sequences and feature matrices) that may be immediately processed with embedding computation techniques known from other fields (such as Jeffrey Pennington, Richard Socher, and Christopher D Manning. "Glove: Global Vectors for Word Representation." In: EMNLP. Vol. 14. 2014, pp. 1532-43 and Tomas Mikolov et al. "Distributed representations of words and phrases and their compositionality". In: Advances in neural information processing systems. 2013, pp. 3111-3119).

Existing methods for (knowledge) graph embeddings like Qiong Yang, Mo Chen, and Xiaoou Tang. "Directed graph embedding". In: Proceedings of IJCAI'07. IJCAI, 2007, Node2Vec (Aditya Grover and Jure Leskovec. "Node2Vec: Scalable Feature Learning for Networks". In: Proceedings of the 22Nd ACM SIGKDD International Conference on Knowledge Discovery and Data Mining. KDD '16. San Francisco, California, USA: ACM, 2016, pp. 855-864. ISBN: 978-1-4503-4232-2. DOI:10.1145/2939672.2939754.URL: http://doi.acm.org/10.1145/2939672.2939754) or RDF2vec (Petar Ristoski and Heiko Paulheim. "RDF2vec: RDF graph embeddings for data mining". In: International Semantic Web Conference. Springer. 2016, pp. 498-514) focus on pure graphs or multigraphs with labelled relations (i.e., subject-predicate-object statements in RDF). They cannot cope with knowledge graphs with statements representing both 2-way and 3-way or even higher arity relationships, even though such cases are common in many areas (for instance in life sciences, where relevant data may come from knowledge bases of different arities, such as protein interaction graphs and pathway hypergraphs).

A very recent method (Jianfeng Wen et al. "On the Representation and Embedding of Knowledge Bases Beyond Binary Relations". In: Proceedings of IJCAI'16. IJCAI/AAAI Press, 2016, pp. 1300-1307) can compute embeddings of knowledge graphs with relations of arbitrary arities, but has limited expressivity since it only targets the semantics of n-ary relations in the Freebase dataset which is only a subset of all possible n-ary relations. Other limits of Jianfeng et al related to this invention concern directionality of the relations. It essentially treats the k-way relationships as undirected in the learning phase. However, an ideal method should distinguish between directed and undirected relationships and support both directed and undirected connections within one hyperedge.

One challenge in this context is the complexity of the higher-arity structures (such as graphs with typed multi-edges or hypergraphs). Their implementations have to carefully balance expressive power and computational efficiency. If edges of mixed directions are to be represented and processed as well, the technical complexity of the required solutions grows even more. Mathematical foundations have to be carefully defined as no standard approaches exist at the moment, and the technical implementation of these foundations must handle the representational overhead (when compared to standard graphs) in a tractable way, which is not trivial.

Invention embodiments may represent the higher-arity (more than binary) and mixed directionality knowledge graphs in a uniform manner by:

(a) Transformation of every (any) input knowledge graph into a hypergraph representation that may have both directed and undirected weighted labelled edges between any number of nodes higher than one. This is the most general graph structure that may accommodate any type of knowledge graphs one may deal with. It also supports different hyperedge cardinalities and thus also non-uniform knowledge graphs, addressing the key point of the technical problem.

(b) Representation of the hypergraph as a bipartite graph where the partitions correspond to vertices and hyperedges, respectively. Invention embodiments may include definition of a set of principles that allow for efficient implementations of this structure in the form of computer programs that satisfy the main purpose of the representation - allowing for uniform sampling of heterogeneous knowledge graphs using a common, well-defined underlying structure.

Then invention embodiments provide methods for:

(a) Sampling of the bipartite / hypergraph representations of the knowledge graph as explained in more detail later using a traversal (e.g., breadth-first search, random walk or A* search). The bipartite representation allows for uniform implementation of traversal for a diverse range of graph-like structures (standard graphs, multigraphs or hypergraphs; cf. formal definitions later on for details). It is enabled by and dependent on the specific principles defined for the bipartite representation. These principles ensure that a traversal executed on the bipartite representation corresponds to a valid traversal with the same properties on the original knowledge graph representation.

(b) Generation of vertex and edge sequences from the traversals. This makes sure that the entities (vertices) and relations (edges) representations are distinguished in the samples.

Finally, invention embodiments compute vector representations of the entities and relations in knowledge graphs. To do that, distributional patterns in the sequences may be analysed by:

a. Sliding a context window over the sampled sequences and computing local entity-entity, entity-relation and relation-relation co-occurrence scores based on the distance within the window.

b. Updating global co-occurrence scores.

c. Optional frequency-based feature selection.

These steps produce a matrix that encodes distributional features of every entity and relation in the knowledge graph. Multiple such feature matrices may be generated from the same knowledge graph by simply adjusting the traversal strategy used for the sampling. The intermediate sequence sets may be processed by algorithms like word2vec (Tomas Mikolov et al. "Distributed representations of words and phrases and their compositionality". In: Advances in neural information processing systems. 2013, pp. 3111-3119). The distributional feature matrices may be treated as embeddings themselves, or straightforwardly processed by more sophisticated embedding learning models like GloVe (Jeffrey Pennington, Richard Socher, and Christopher D Manning. "Glove: Global Vectors for Word Representation." In: EMNLP. Vol. 14. 2014, pp. 1532-43). This flexibility is useful for the ultimate effect invention embodiments - the ability to easily adjust different existing embedding techniques to knowledge graph structures with varying arities and mixed directionality.

The computed feature matrices confidently represent the complex structure of the original knowledge graphs as they are based on well-understood mechanisms of graph sampling. Semantics of the entities and relations is naturally preserved in the sampling process. The matrices may be directly used for producing entity and/or relation embeddings using state of the art or newly developed techniques utilising vector space representations.

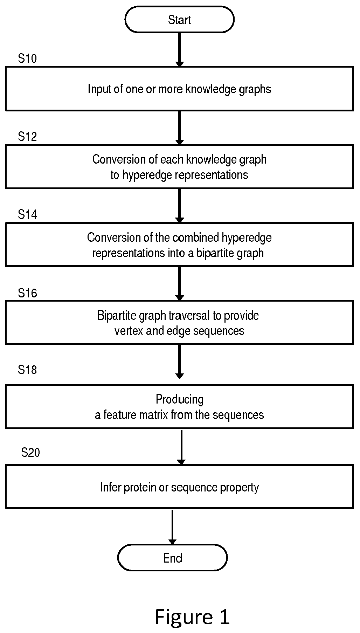

Figure 1 is an overview flowchart of an invention embodiment. Step S10 inputs two or more knowledge graphs related to proteins in the form of vertices and edges and having different arities, at least one of the knowledge graphs being directional and at least one of the knowledge graphs being labelled. The directional graph or graphs may be partially directional (with only some edges having directions). The same graph may be both directional and labelled. The graphs may be input in any machine-readable form, such as in a list of vertices and associated edges. Embodiments may use CSV input, or alternatively triples or quads in RDF format, or formats for representing structured data (such as GraphML).

Step S12 converts each of the knowledge graphs to a hyperedge representation. This may be a list of unique edge identifiers, each with any source and target vertex vertices, any vertices connected by the edge in an undirected manner, and any labels or weights associated with the edge. Hence in this representation, each graph may consist of many unique edge identifiers in a list.

Step S14 converts the hyperedge representations into a bipartite graph with the vertices and labels as entities in one set and linked to the edges as entities in the other set by notional auxiliary edges A. Each notional auxiliary edge links a vertex or a label to an edge of the graph to which it is joined/applied in the hyperedge representation. Hence in the bipartite graph (and in the following processing) each edge and label as well as each vertex is an entity in the graph, joined by edges A.

The hyperedge representations may be combined into one before conversion. Alternatively, each hyperedge representation may be made into a bipartite graph and the bipartite graphs subsequently joined. In either case, for the combination, nodes with the same name (or those considered to have the same name using a mapping tool) are considered as the same node.

Step S16 traverses the bipartite graph to provide possible sequences along the links between the sets. For example, one strategy might find paths in the graph. This strategy may start at each node in turn (or a random node) and explore possible paths along the bipartite graph of a specified length, or the full path length. Various known mechanisms may be used to explore the full graph, such as depth-first, breadth-first or best-first search. In the end result, and in the absence of weighting parameters, if there are two or more edges from a vertex, then the resultant paths should be split evenly between those edges. The paths are used as sequences.

Step S18 produces a feature matrix of the vertices, labels and edges, with a score, such as a co-occurrence score, in each cell of the feature matrix. This feature matrix may be a square matrix of all the entities, which is initialised to zero. It may be populated using the sequences. For example, values derived from the sequences may be added to the matrix, sequence by sequence, for instance based at least partially on the proximity of the entities (node and edges from the originally input graphs) in the sequence. A context window (shorter than the path length) may start at each point/entity in the sequence but the last (since a path needs two entities as a minimum) to give context sequences. The co-occurrence scores between each starting point and the subsequent entities in the context sequence are then calculated, taking into account the distance between the entities in the context sequence. The feature matrix is created as co-occurrence scores for each sequence are added.

Finally, step S20 uses the feature matrix to infer a property of a protein (or other biomedical entity/graph item) or a sequence in the knowledge graph based on the (co-occurrence) scores. For example, the cosine similarity of two proteins (calculated on the basis of all the nodes and vertices in the combination of input graphs) may be inferred.

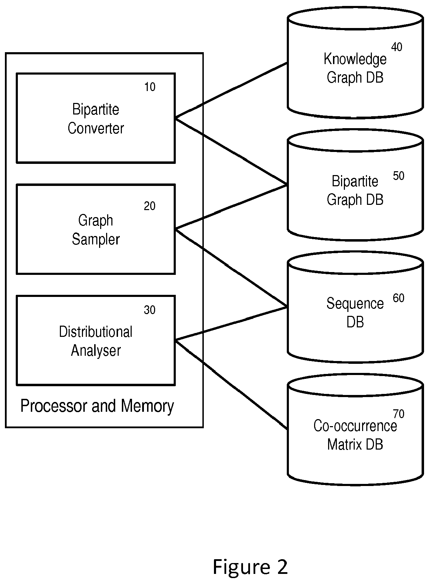

Figure 2 shows the computational units for the functions carried out and explained above: a bipartite converter 10, a graph sampler 20 and a distributional analyser 30. These are all shown in a processor and memory block. The bipartite converter receives two or more knowledge graphs (e.g., from knowledge graph database or DB 40) related to proteins in the form of vertices and edges and having different arities, at least one of the knowledge graphs being directional and at least one of the knowledge graphs being labelled. The input may be in any suitable form. The bipartite converter first converts each of the knowledge graphs to a hyperedge representation. This may be as a list of unique edge identifiers, each with any source and target vertex vertices, any vertices connected by the edge in an undirected manner, and any labels associated with the edge. From the hyperedge representation, there is a conversion into a bipartite graph with the vertices and labels in one set and the edges in the other set. The vertices and labels are linked to the edges as per the hyperedge representations by auxiliary edges A. This bipartite graph may be saved in bipartite graph DB 50, and/or in local memory.

The graph sampler 20 traverses the bipartite graph to provide possible sequences along the links between the sets, as explained in more detail later. Sufficient sequences are produced to explore the graph. The sequences produced may be saved in sequence DB 60, and/or in local memory.

The distributional analyser 30 produces a feature matrix of the vertices, labels and edges from the sequences, with a co-occurrence score in each cell of the feature matrix; and uses the feature matrix to infer a property of a protein in the knowledge graph based on the co-occurrence scores. The co-occurrence matrix and further results may be stored in local memory and potentially also in the co-occurrence matrix DB 70.

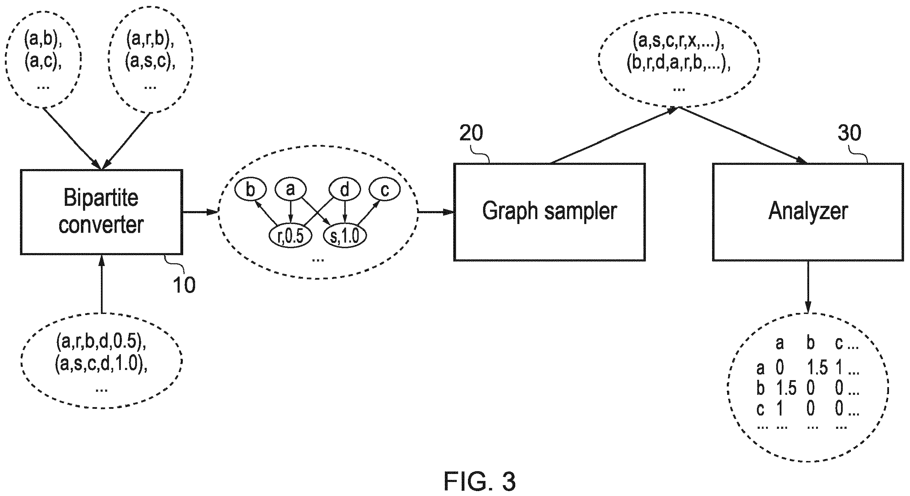

The system according to one embodiment as a whole and its inputs is illustrated in Figure 3. The bipartite converter 10 ingests knowledge graphs (of varying arities and directionality) and outputs a corresponding bipartite representation. This may then be used for running a traversal-based sampling algorithm by the graph sampler 20. The result of this step are sequences of entities and relations in the knowledge graph. These may be used for producing embeddings by an external tool, or passed on to an internal distributional analyzer module 30. This component computes vector space representations of the entities and relations in the knowledge graph (i.e., a distributional feature matrix). This may be used either as a rudimentary embedding, or processed further by external embedding tools. The same module may be used to infer properties.

The details of the particular high-level modules are described in the following sections.

**Bipartite Conversion**

This section first introduces the core structure used for the extensible representation and then describes rules for converting selected graph structures into the universal representation. Finally, illustrative examples of selected graph structures and their transformed representations are provided.

**Bipartite Representation of Graph-Like Structures**

As a basis for the representation of graph structures (standard graphs, multigraphs and hypergraphs in particular), the inventors have chosen bipartite graphs defined as

$$(V, E, A)$$

where $V, E$ are node partitions that correspond to vertices and (hyper)edges, respectively, in the graph structure being represented by the bipartite graph. $A \subseteq V \times \mathcal{O} \times E$ is a set of directed or undirected auxiliary edges between the nodes in the $V, E$ partitions, where $\mathcal{O} = \{\leftarrow, \rightarrow, \leftrightarrow\}$ is a set of possible edge orientations (from $V$ to $E$, from $E$ to $V$ or undirected, respectively). The edges in $A$ emulate the actual edges or hyperedges in the graph structure being represented.

To increase the expressive power of the base bipartite representation, several mappings associated with the bipartite graph node partitions may be defined:

$$\lambda_v : V \rightarrow L_v, \ \lambda_e : E \rightarrow L_e, \ \lambda_w : E \rightarrow \mathbb{R},$$

where $Lv, Le$ are sets of vertex and edge labels, respectively. The mappings are primarily meant to associate the particular vertex and edge nodes with types and weights that are required for appropriate representation of general, possibly labelled and weighted graph structures. If not specified otherwise, the following default settings of the labels are defined:

$$L_v = \{0_v\}, \forall u \in V : \lambda_v(u) = 0_v, L_e = \{0_e\}, \forall e \in E : \lambda_v(e) = 0_e, \forall e \in E : \lambda_w(e) = 1,$$

where *0v, 0e* are default singleton labels (only one value) for the vertex and edge nodes, respectively, from the set of real numbers. The bipartite representation allows for uniform representation of various graph structures of different levels of expressivity and complexity. There is a price for this, though, in terms of increase space and time complexity of the standard graph operations executed via the bipartite representation. The increase is generally linear and thus does not mean a shift to another complexity class, but even a linear factor increase may be difficult in many applications on large data. Therefore the following specific optimisations of the bipartite representation may be introduced:

- Integer encoding of all entity and relation labels so that the vertices of the bipartite graph can be stored more efficiently (i.e., using a fixed size integer instead of variable size string type).

- Representation of the edges in $A$ using sparse Boolean matrices which allows for efficient retrieval of node successors, predecessors and adjacent nodes by means of optimised matrix operations.

The three types of graph structures of primary interest in this field are standard graphs, edge-labelled multigraphs and hypergraphs. For each of these, specific restrictions on their bipartite representation, may be imposed as outlined in the following paragraphs.

**Standard graphs** (I) For all $e \in E$, $d(e)$ is 2, where $d(u)$ is a degree of the node $u$ in the bipartite representation. If the graph being represented is directed, $d_i(e) = d_o(e) = 1$ for every $e \in E$, where $d_i(u), d_o(u)$ is the in-degree and out-degree, respectively, for the node $u$ in the bipartite representation *(no hyperedges; no edge "sinks" or "springs")*. (II) If $(v_i, e_a), (v_j, e_a) \in A$ and $(v_i, e_b), (v_j, e_b) \in A$, then $e_a = e_b$ *(no multiedges)*. (III) $A$ is a set *(no loops)*.

**Edge-labelled multigraphs** (I) For all $e \in E$, $d(e)$ is 2. If the graph being represented is directed, $d_i(e) = d_o(e) = 1$ for every $e \in E$ *(no hyperedges; no edge "sinks" or "springs")*. (II) $A$ can be a multiset *(loops are possible)*. (III) If $(v_i, e_a), (v_j, e_a) \in A$ and $(v_i, e_b), (v_j, e_b) \in A$, then $\lambda_e(e_a) \neq \lambda_e(e_b)$ *(no multiedges sharing the same label)*.

**Hypergraphs** (I) For all $e \in E$, $d(e) > 1$. If the hypergraph being represented has directed relations, then $d_i(e) > 0$, $d_o(e) > 0$ for all $e \in E$ *(no hyperedge "sinks" or "springs")*. (II) $A$ can be a multiset *(loops are possible)*. (III) If $(v_i, e_a), (v_j, e_a) \in A$ and $(v_i, e_b), (v_j, e_b) \in A$, then $\lambda_e(e_a) \neq \lambda_e(e_b)$ *(no hyperedges sharing the same label)*.

Note that the multiset possibility for multigraphs and hypergraphs only makes sense for undirected relationships, since directed loops can be represented even if $A$ is a set. No particular restrictions are imposed on the $\lambda_v$ label mapping as those labels as the interpretation of the vertex node labels is left for the algorithms processing the graph structures.

**Transformation of Graph-Like Structures into Bipartite Representations**

The crucial instrument for the transformation is a generalised (hyper)edge defined as a tuple

$$(e, S, T, U),$$

where $e$ is a unique edge identifier and $S$, $T$ are sets of source and target nodes, respectively, that are connected by the edge in a directed manner. $U$ is a set of nodes that are connected by the edge in an undirected manner. The $S$, $T$, $U$ sets can satisfy the following conditions: (I) $|S \cup T \cup U| > 1$ *(no unary or nullary relations)*. (II) $S \cap T = S \cap U = S \cap U = \emptyset$ *(unique vertex roles)*. (III) If $U \neq \emptyset$, then either $S,T = \emptyset$ or $|S| \neq \emptyset \wedge |T| \neq \emptyset$ *(only hypergraphs can be of mixed directionality)*.

To illustrate this definition, consider an undirected edge $e$ connecting nodes $a$, $b$ in a standard graph. Such an edge is represented as $(e, \emptyset, \emptyset, \{a,b\})$. A directed edge $f$ leading from $a$ to $b$ would be represented as $(f, \{a\}, \{b\}, \emptyset)$. Hyperedges can naturally be represented as $(\_, S, T, U)$ where $|S \cup T \cup U| > 2$. Weighted and typed (labelled) edges can be represented by introducing corresponding mappings on the edge set and/or unique edge identifiers.

Assuming an original graph structure representation consisting of vertices $\overline{V}$ and edges $\overline{E}$, the corresponding bipartite representation $(V,E,A)$ is constructed as follows:

- $V$ is equal to $\overline{V}$.
- For every (hyper)edge $(e, S, T, U)$, $\in \overline{E}$ there is a node $e \in E$.
- Every (hyper)edge $(e, S, T, U)$, $\in \overline{E}$ corresponds to the following edges in A: $\{(u, \rightarrow, e) | u \in S\} \cup \{(u, \leftarrow, e) | u \in T\} \cup \{(u \leftrightarrow, e) | u \in U\}$.
- If there are any mappings (or labels/weights) associated with the original representation, they are

preserved in the $\lambda_v$, $\lambda_e$, $\lambda_w$ mappings accordingly.

**Examples**

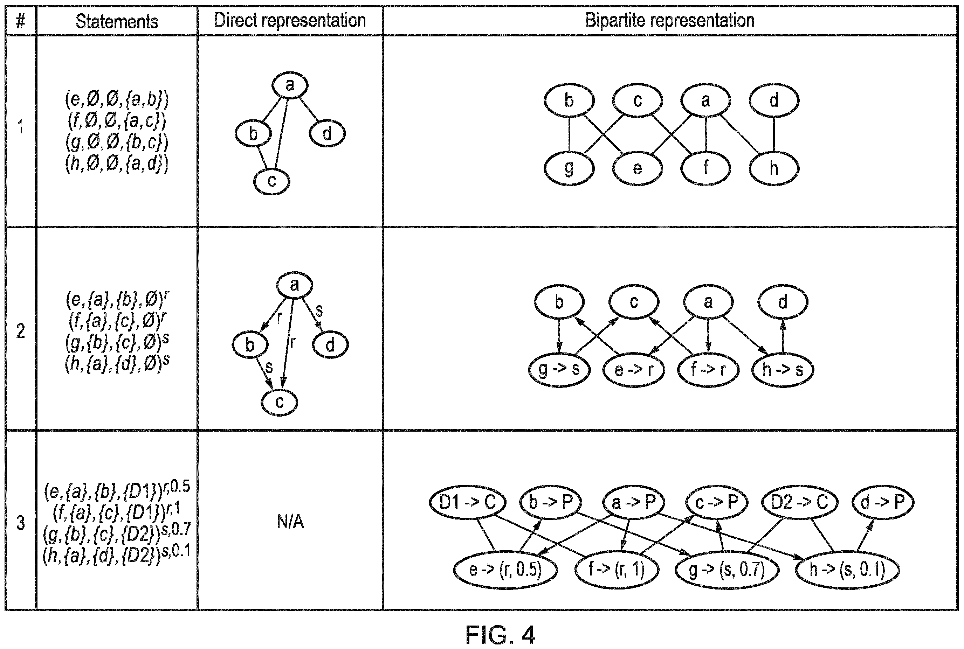

Figure 4 shows simple examples of sets of statements encoding a standard undirected graph, a directed multigraph and a mixed weighted hypergraph, and the corresponding bipartite representations. For reference, direct graphical representations of the graph and multigraph are shown. Due to the relative complexity of the depiction of higher-arity relationships, a direct graphical representation of the hypergraph is not given here to keep the example concise and comprehensible.

The statements correspond to relations (*i.e.* edges, multiedges or hyperedges) in the graph structures and are presented in the notation introduced before. The statements may optionally be associated with relation types and/or real weights. These are given in the superscript of the corresponding edge representations where applicable.

The statements in Example 1 correspond to an unlabelled, undirected standard graph. The $V$, $E$ nodes are $\{a,b,c,d\}$ and $\{e,f,g,h\}$, respectively. The edges in $A$ emulate the edges between nodes in $V$ via 2-hop paths connecting two nodes in $V$ through one node in $E$. For instance, the $(a,b)$ edge in the original graph corresponds to the $(a,e,b)$ path. Example 2 presents a directed multigraph - a graph of binary relations where the relations are labelled by their types and more relations of different types may exist between the same two nodes. Similarly to the previous example, the edges in the original structure are emulated by 2-hop paths connecting two nodes in $V$ through one node in $E$. The difference is that the edges in $A$ are now directed to allow for representation of the original relations' direction. Additionally, there is also a $\lambda_e$ labelling associating the $E$ nodes with the corresponding predicate labels, *i.e.* relation types.

Example 3 presents the most expressive graph structure - a hypergraph where more than 2 nodes may be connected by a weighted edge, with both node and edge type labelling present. The $\lambda_e$, $\lambda_w$ labels are reflected by the edge mapping pairs in the *E* nodes in the bipartite representation - for instance, the fact that the statement *e* has a predicate (label/type) *r* and weight *0.5* is reflected by the *(r, 0.5)* pair in the corresponding *E* node label. The *V* nodes are labelled as well in this example, to distinguish between primary arguments *(P)* and context arguments *(C)* of the relationships. Primary arguments may be for instance proteins, genes, chemicals, etc., occurring in relations in documents, while the documents themselves may be context arguments. The directionality of the bipartite representation is mixed. This is to model the difference between the semantics of the primary and context arguments (the context arguments like documents may hardly be associated with any particular directionality with respect to the primary arguments), and to allow for both directed and undirected relations in the same model.

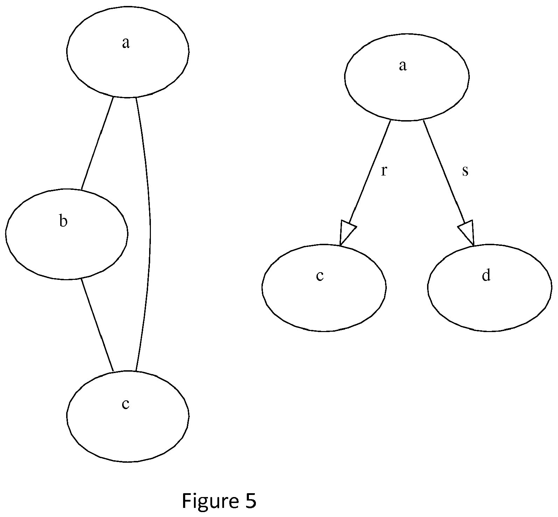

Regarding merging graph structures with different arities and mixed directionality of edges, two structures depicted in Figure 5 are assumed. One is a standard undirected graph and the other is a binary multigraph with typed relations. Assume the hyperedge representations for the standard graph and multigraph are

$$(e, \emptyset, \emptyset, \{a, b\}), (f, \emptyset, \emptyset, \{a, c\}), (g, \emptyset, \emptyset, \{b, c\}),$$

and

$$(i, \{a\}, \{c\}, \emptyset)^r, (j, \{a\}, \{d\}, \emptyset)^s,$$

respectively.

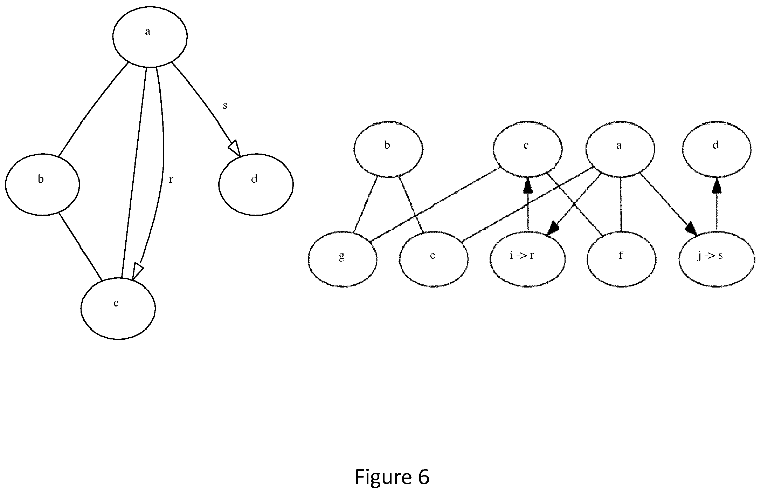

Then the merge of these structures and its corresponding hypergraph representation are as presented in Figure 6.

## Uniform Sampling

This section first describes the correspondence between paths in the original graph structures and the bipartite representations, which is then used to define traversals that may be used for sampling the structures. Examples of sampling are then given.

## Paths

In any of the graph structures considered (*i.e.* standard graphs, multigraphs and hypergraphs), paths may be defined using the notions introduced in Adrian Bondy and Uppaluri SR Murty. Graph Theory (Graduate Texts in Mathematics 244). Springer, 2008. *A path* is a simple graph (a binary graph with no loops or multiedges) that may be ordered in a linear sequence of nodes so that two nodes are adjacent if they are consecutive in the sequence.

Thus, a path of length *k* (i.e. having *k* nodes) may be represented as a sequence

$$(v_1, e_1, v_2, e_2, \ldots, v_{k-1}, e_{k-1}, v_k)$$

where $v_i, e_j, i \in \{1,...,k\}, j \in \{1,...,k - 1\}$ are nodes and (hyper)edges in the corresponding graph structure. Obviously, an edge $e_z$ may appear in a path sequence between nodes $v_x$ and $v_y$ if and only if $v_x, v_y$ are source and target nodes of the $e_z$ edge, respectively.

Every path of length *k* in the original graph structure representation corresponds to a path of length *2k-1* in the bipartite representation. The bipartite representation of a path

$$(v_1, e_1, v_2, e_2, \ldots, v_{k-1}, e_{k-1}, v_k)$$

is a sequence

$$(v_1, a_1, e_1, a_2, v_2, a_3, e_2, a_4, \ldots, a_{2k-3}, v_{k-1}, a_{2k-2}, e_{k-1}, a_{2k-1}, v_k)$$

where $v_x \in A, e_y \in E, a_z \in A$ for $x \in \{1,...,k\}, y \in \{1,..., k- 1\}, z \in \{1,...,2k - 1\}$. For any sub-path $v_i, a_j, e_k, a_l, v_m$ in the path sequence, the following has to be satisfied: $a_j = (v_i, o_l, e_k), a_l = (v_m, o_2, e_k)$, *where* $o_1 \in \{\rightarrow, \leftrightarrow\}$ *and* $o_2 \in \{\leftarrow, \leftrightarrow\}$. Due to direct correspondence between the paths in the original structures and their bipartite representations, reference is to the simpler notation consisting only from the *V,E* elements unless specified otherwise. However, in terms of properties of the nodes, we will refer to the bipartite representation, unless specified otherwise.

The next paragraphs review several possible options for traversing the graph structures uniformly by means of their bipartite representations. Special attention is paid to random walks as their implementation using the specific bipartite representation proposed requires a formal specification.

**Search-Based Traversals** Traversals based on depth-first, breadth-first or best-first search are all based on transitions between nodes using a specific heuristic that is independent of the bipartite representation. The transitions themselves, however, are influenced by it in the following manner. The transition from a node *u* to a node *v* via an edgee

$$(u, e, v)$$

in a graph structure corresponds to a transition

$$(u, a_{u,e}, e, a_{e,v}, v)$$

in the corresponding bipartite representation, where $a_{u,e}, a_{e,v}$ are the auxiliary edges connecting the bipartite nodes corresponding to the *u, v* entities and the *e* relation. Sequences generated by search-based traversals may be directly obtained as contiguous sub-paths of the path resulting from the search.

**Random Walks** Random walks have been identified as an efficient and universally applicable traversal-based method for sampling graph data (see Jure Leskovec and Christos Faloutsos. "Sampling from large graphs". In: Proceedings of the 12th ACM SIGKDD international conference on Knowledge discovery and data mining. ACM. 2006, pp. 631-636). A random walk in any of the graph structures considered may be conveniently defined within the corresponding bipartite representation. A random walk is defined as a path (possibly infinite) in which the transition in the sequence is determined randomly, based on the properties of the current node and its associated outgoing edges (in the bipartite representation).

Consider a single transition $(v_x, e_x, v_x+1)$ from a vertex node $v_x$ to $v_x+1$ via an edge node $e_x$. The probability $p[(v_x, e_x, v_x+1)]$ of that transition is a joint probability of two events: 1. probability $p(e_x|v_x)$ of getting from the vertex node $v_x$ to the edge node $e_x$; 2. probability $p(v_x+1|e_x)$ of getting from the edge node $e_x$ to the vertex node $v_x+1$. As these two events are independent, the transition probability is $p[(v_x, e_x, v_x+1)]=p(e_x|v_x)p(v_x+1|e_x)$.

The $p(e_x|v_x)$ probability is

$$p(e_x|v_x) = \frac{\lambda_w(e_x)}{\sum_{e \in E_o(v_x)} \lambda_w(e)},$$

where $E_o(v_x) = \{e|e \in E \wedge \exists a \in A : a = (v_x, \rightarrow, e) \, Va = (v_x, \leftrightarrow, e)\}$ is the set of outgoing edge nodes with respect to $v_x$. The $p(v_x+1|e_x)$ probability is zero if $v_x+1 = v_x$ or $v_x+1 \notin V_o(e_x)$, where $V_o(e_x) = \{v|v \in V \wedge \exists a \in A : a = (v, \leftarrow, e_x) \, V_a = (v, \leftrightarrow, e_x)\}$ is the set of outgoing vertex nodes with respect to $e_x$. If $v_x+1 \in V_o(e_x) \setminus \{v_x\}$, the $p(v_x+1|e_x)$ probability is

$$p(v_{x+1}|e_x) = \frac{1}{|V_o(e_x) \setminus \{v_x\}|},$$

*i.e.* proportional to the out-degree of the edge node $e_x$, excluding the starting node of the transition. The joint transition probability is thus

$$p[(v_x, e_x, v_{x+1})] = \frac{\lambda_w(e_x)}{|V_o(e_x) \setminus \{v_x\}| \sum_{e \in E_o(v_x) \lambda_w} \lambda_w(e)}.$$

Sequences generated by the random walk traversals may be directly obtained as contiguous sub-paths of the random walks (or even the walks themselves).

**Examples**

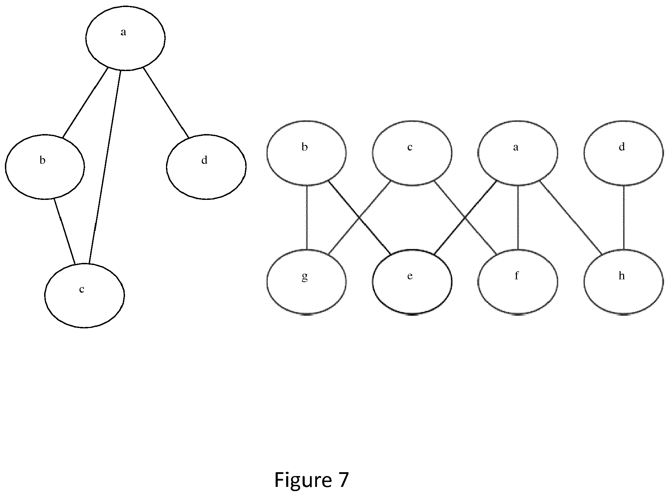

To illustrate the notions of path and traversal, take Examples #1 and #3 from Figure 4 and traversal by means of random walk (that is perhaps least trivial of the possibilities reviewed in the previous section). Example #1 presents a standard graph *G* with direct and bipartite graphical representations given in Figure 7.

A path in the graph *G* is for instance the sequence

$$P_g = (b, g, c, f, a, h, d).$$

Let *W(u, k, G)* be a random walk of length *k* starting in the node *u* of the graph *G*. The probability $p[P_g = W(b,4,G)]$ that a random walk of length *4* starting in the node *b* in *G* will be equivalent to the path $P_g$ is

$$p[P_g = W(b, 4, G)] = p[(b, g, c)]p[(c, f, a)]p[(a, h, d)] = \frac{1}{2} \cdot \frac{1}{2} \cdot \frac{1}{3} = 0.08\bar{3},$$

*i.e.* product of probabilities of the transitions that would produce $P_g$ by random after starting in *b*. Note that as there are no edge weights defined, the default weight of 1 is used and thus the transition probabilities effectively depend only on the degrees of the (edge) nodes along the path.

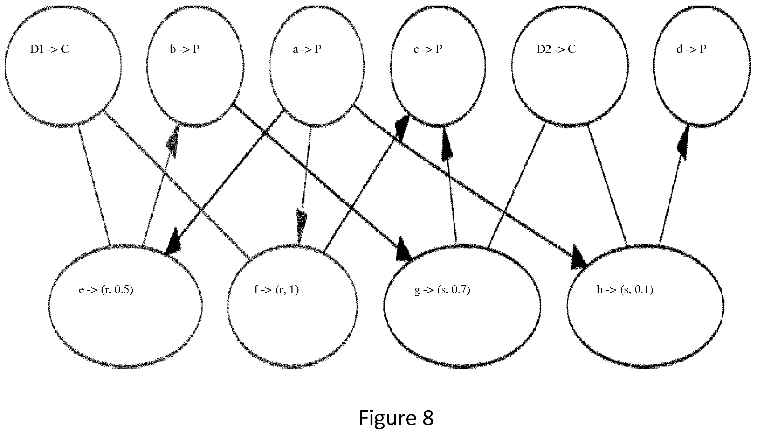

Example #3 in Figure 4 presents an expressive hypergraph *H* with bipartite graphical representation given in Figure 8.

An example of a path in *H* is

$$P_h = (a, f, D1, e, b).$$

The probability $p[P_h = W(a,3,H)]$ that a random walk of length 3 starting in the node a in *H* will be equivalent to the path $P_h$ is

$$p[P_h = W(a, 3, H)] = p[(a, f, D1)]p[(D1, e, b)] = (\frac{1}{0.5 + 1 + 0.1} \cdot \frac{1}{2})(\frac{0.5}{0.5 + 1} \cdot \frac{1}{1}) = 0.10416$$

All random walks generated in the examples above may be directly used as a sample sequence of the corresponding graph structures.

**Distributional Vectorization**

This section describes how the sequences produced by sampling the graph structures may be used for constructing distributional feature vectors of entities and relationships. Examples are then given.

**From Sequences to Vectors**

Assume an empty (*i.e.,* zero) feature matrix *F* with |*E*| + |*V*| rows and columns that corresponds to a graph structure *G* with nodes *V* and (hyper)edges *E*. Denote the element corresponding to the row $s \in E \cup V$ and the column $s' \in E \cup V$ as *F(s,s')*.

The distributional patterns in each sequence in the form

$$s = (v_1, e_1, v_2, e_2, \ldots, v_{k-1}, e_{k-1}, v_k)$$

sampled from the graph structure *G* (the length of sis 2*k*-1) are then used for updating the feature matrix *F* as follows:

- Initialise a local co-occurrence weight mapping $w_{cooc} : (E \cup V) \times (E \cup V) \to \mathbb{R}$ to zero for all key values.
- For each context window of size *c*, exhaustively iterating over the starting positions

$$s_i \in \{s_1, s_2, \ldots, s_{2k-2}\}$$

in the sequence *s*, do:

- Take $s_i$ as a pivot for which the local co-occurrence weights are computed.
- For each $j \in \{i + 1, i + 2, \ldots, \max(c, 2k - 1)\}$ update the local co-occurrence as follows:

$$* \text{ If } j - i \leq \alpha \text{ then } w_{cooc}(s_i, s_j) \leftarrow w_{cooc}(s_i, s_j) + 1;$$
$$* \text{ Else, } w_{cooc}(s_i, s_j) \leftarrow w_{cooc}(s_i, s_j) + (j - i - \alpha + 1)^{-\beta}.$$

- For each $s, s' \in E \cup V$, update the feature matrix *F* as follows:

$$- F(s, s') \leftarrow F(s, s') + w_{cooc}(s, s')$$
$$- F(s, s') \leftarrow F(s, s') + w_{cooc}(s', s)$$

The result of this process is a symmetric square matrix that encodes each entity and relation as a vector of weights that reflect their co-occurrence with other entities and relations in the sampled sequences. The way how the co-occurrence weights are computed may be influenced by three parameters:

- *c* - a parameter defining the context window size, defining how many consecutive elements at a time are considered for a non-zero co-occurrence weight. This may be seen as an analogue of natural sentence boundaries in co-occurrence computations for natural language texts.

- $\alpha$ - a parameter defining immediate neighbourhood, *i.e.,* a distance within which elements are considered very closely related (receiving local co-occurrence weight of 1, the highest possible). This is primarily to reflect that if two entities are directly linked by a relation, they should intuitively be strongly associated even though they are two positions apart in the sampled sequence (with the linking relation in between).

- $\beta$ - a parameter defining the damping of local co-occurrence weights as a function of distance from the pivot. This is to reflect that elements that are further apart should be less strongly associated.

Optional feature selection may be achieved by pre-processing the sequences before computing the distributional feature matrix and storing their frequency or other characteristics applicable to feature matrix and storing their frequency or other characteristics applicable to feature selection in an analogical manner to traditional text mining. Examples of characteristics to store are relative frequencies of elements in the sequences or their inverse sequence frequency (analogical to inverse document frequency in text processing). The values of the characteristics may then be used for deciding whether a certain element should be considered for updating the co-occurrence scores when encountering it in the sliding context windows. For instance, one may decide not to reflect any co-occurrences with elements that occur less than five times in all sequences, or that occur in all sequences.

**Examples**

Consider the path

$$P_g = (b, g, c, f, a, h, d).$$

from the previous example as a sampled sequence. Further assume the following parameter values: $c = 5$, $\alpha = 2$, $\beta = 1$. Then the co-occurrence scores corresponding to the $P_g$ sequence are computed as follows. There are six context windows:

- (*b,g,c,fa*),
- (*g,c,f,a,h*),

- (*c,fa,h,d*),
- (*f,a,h,d*),
- (*α,h,d*),
- (*h,d*).

The $w_{cooc}$ values for (*b,g*), (*b,c*) are equal to 1 since the distance between the elements in the sequence is below or equal to 2 (the α parameter). The values for (*b,f*), (b,a) are then $(4-1-2+1)^{-1} = 0.5$, $5-1-2+1)^{-1} = 0.\overline{3}$. pairs from all context windows are $w_{cooc}(g,c) = w_{cooc}(g,f) = w_{cooc}(c,f) = w_{cooc}(c,a) = w_{cooc}(f,a) = w_{cooc}(f,h) = w_{cooc}(a,h) = w_{cooc}(a,d)$ $= \underline{w}_{cooc}(h,d) = 1$, $w_{cooc}(g,a) = w_{cooc}(c,h) = w_{cooc}(f,d) = 0.5$, $w_{cooc}(g,h) = w_{cooc}(c,d) = 0.\overline{3}$.

Assuming the rows and columns corresponding to the *a,b,c,d,e,f,g,h* elements, the resulting co-occurrence feature matrix is:

$$F = \begin{array}{c} \\ a \\ b \\ c \\ d \\ e \\ f \\ g \\ h \end{array} \begin{array}{cccccccc} a & b & c & d & e & f & g & h \\ \left( \begin{array}{cccccccc} 0 & 0.\overline{3} & 1 & 1 & 0 & 1 & 0.5 & 1 \\ 0.\overline{3} & 0 & 1 & 0 & 0 & 0.5 & 1 & 0 \\ 1 & 1 & 0 & 0.\overline{3} & 0 & 1 & 1 & 0.5 \\ 1 & 0 & 0.\overline{3} & 0 & 0 & 0.5 & 0 & 1 \\ 0 & 0 & 0 & 0 & 0 & 0 & 0 & 0 \\ 1 & 0.5 & 1 & 0.5 & 0 & 0 & 1 & 1 \\ 0.5 & 1 & 1 & 0 & 0 & 1 & 0 & 0.\overline{3} \\ 1 & 0 & 0.5 & 1 & 0 & 1 & 0.\overline{3} & 0 \end{array} \right) \end{array}$$

**Specific Example**

A specific instantiation of the entities and relations in Figure 4, Example #3 is as follows:

a: AKT

b : FOX01

c: BIM

d: XIAP

Relation r: Inhibition
Relation s: activation
Context D1: Inhibition of aptoptosis pathway
Context D2: Activation of survival pathway
Undirected relations are: protein interactions

Nodes e, f, g, h are nodes created in the bipartite representation to materialise relations.
In this example potential protein similarity is discovered based on the related entities and context. This may be used as well to discover similar sequence patterns in the data leading to the discovery of new biological function such as antagonist pathways, etc. cosine similarity may be used, as explained in later examples.

**Worked Examples**

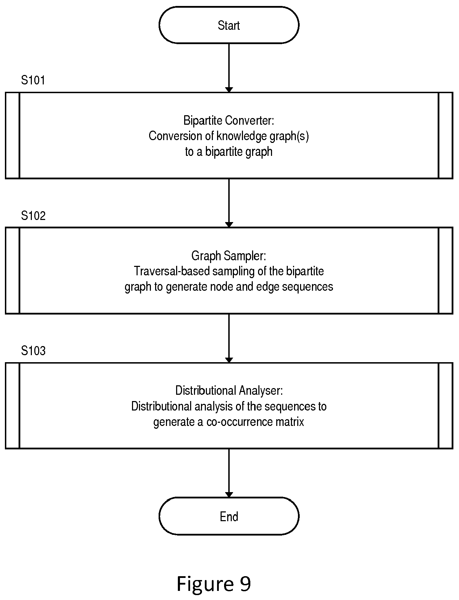

The first worked example considers the first part of the method in two stages: conversion to a bipartite graph as shown in S101 of Figure 9, and graph sampling, as shown in step S102 of Figure 9.

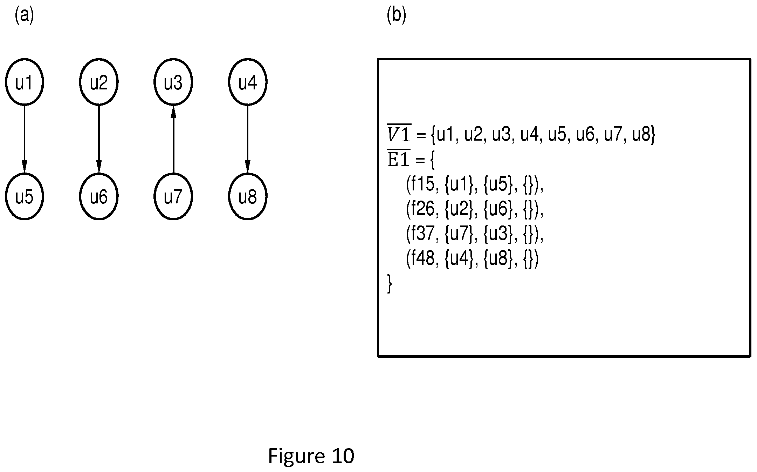

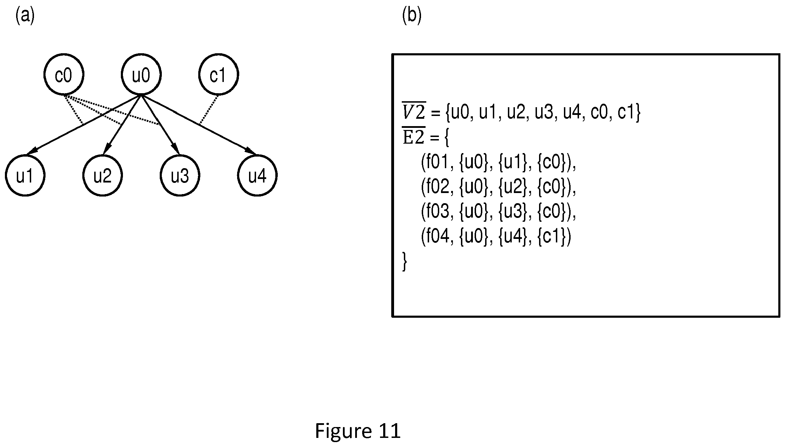

Two graphs shown in Figures 10 and 11 are the starting point.

The standard graph in Figure 10 may be considered as a set of protein interactions and can be read as for example: "protein *u1* interacts with protein *u5*".

A better embedding of proteins *u1* to *u8* would allow identification of similar proteins and then inference of some interactions that are missing from the input graph but of relevance for a biologist trying to model all interactions that could influence a particular disease.

The hypergraph in Figure 11 may be considered as a set of protein interactions happening within a pathway context and can be read this way:

"protein *u0* interacts with protein *u1* in the context of pathway *c0*".

Getting a better embedding of the proteins *u0* to *u4* and of pathways *c0* and *c1* would allow identification of similar proteins (using **extra information of their interaction context**) and then inference of some interactions that are missing from the input graph but of relevance for a biologist trying to model all interactions that could influence a particular disease.

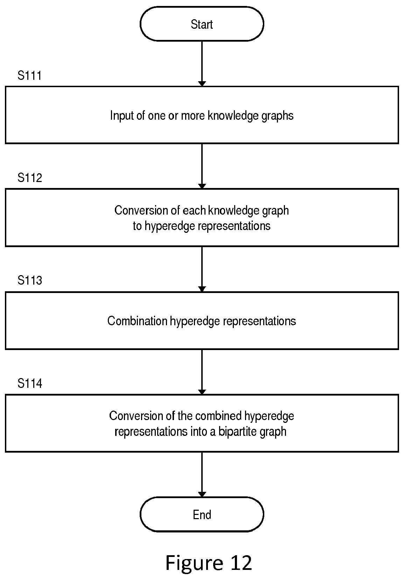

The graphs are processed as shown in Figure 12.

In step S111 the two knowledge graphs in Figure 10a and Figure 11 a are inputted. Figure 10a is a standard graph. In this example the graph consists of 4 parts but a graph may be connected and may consist of only 1 part. The graph may be represented in an arbitrary format (e.g. a list of node pairs). It may be directly represented with hyperedge representations introduced in the next step. Figure 11a is a hypergraph. Each hyperedge connects a source node, a target node, and an additional node with no direction. For example, the leftmost hyperedge connects *u0* (source node), *u1* (target node), and *c0* (additional node). The hypergraph may be represented in an arbitrary format. It may be directly represented with hyperedge representations introduced in the next step.

In step S112 Figure 10a and Figure 11a are converted to Figure 10b and Figure 11b respectively. Each line corresponds to a hyperedge representation *(e, S, T, U)*. Since the leftmost edge in Figure 10a connects *u1* as a source node and *u5* as a target node, S={u1} and T={u5}. Since there is no node connected in an undirected manner, U={}. The hyperedge is named as f15. As a result, (f15, {u1}, {u5}, {}) in Figure 10b is obtained. The other tuples are obtained similarly.

Since the leftmost hyperedge in Figure 11 a connects *u0* as a source node, *u1* as a target node, and *c0* as a node connected in an undirected manner (the edge label is taken as a node), S={u0}, T={u1}, and U={c0}. The hyper edge is named as f01. As a result, *(f01, {u0}, {u1}, {c0})* in Figure 11b is obtained. The other tuples are obtained similarly.

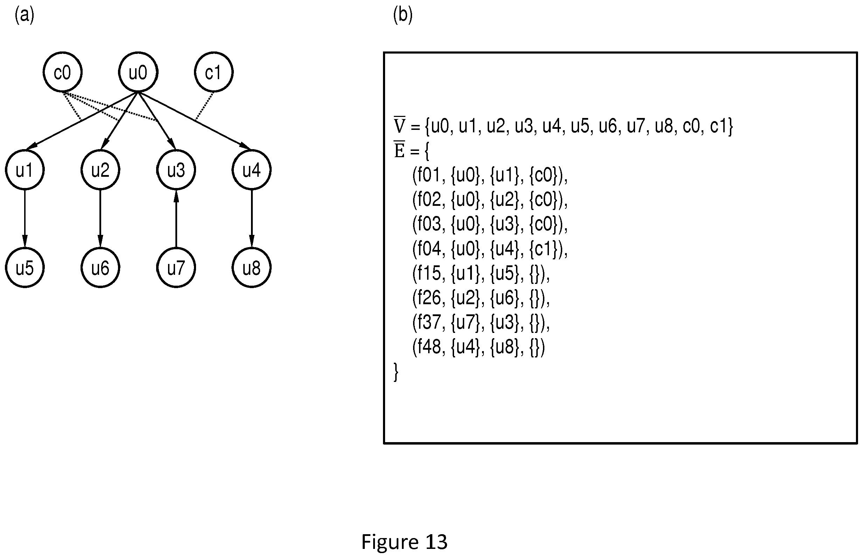

In step S113 the two hyperedge representations in Figure 10b and Figure 11b are combined into Figure 13b. Figure 13a is a visually understandable version of Figure 13b. Here nodes with the same names in the different representations are simply considered as the same nodes. Nodes with different names may be considered as the same nodes, for example by using a node mapping table. A combination operation is performed for hyperedge representations. It may be performed for knowledge graphs or may be performed for the bipartite graphs introduced in Step S114.

The hypergraph In Figure 13 may be considered as a set of protein interactions happening within a pathway context and can be read for example:

"protein *u0* interacts with protein *u1* in the context of pathway *c0*". Getting a better embedding of the proteins *u0* to *u4* and of pathways *c0* and *c1* would allow identification of similar proteins (using extra information of their interaction context) and then inference of some interactions that are missing from the input graph but of relevance for a biologist trying to model all interactions that could influence a particular disease.

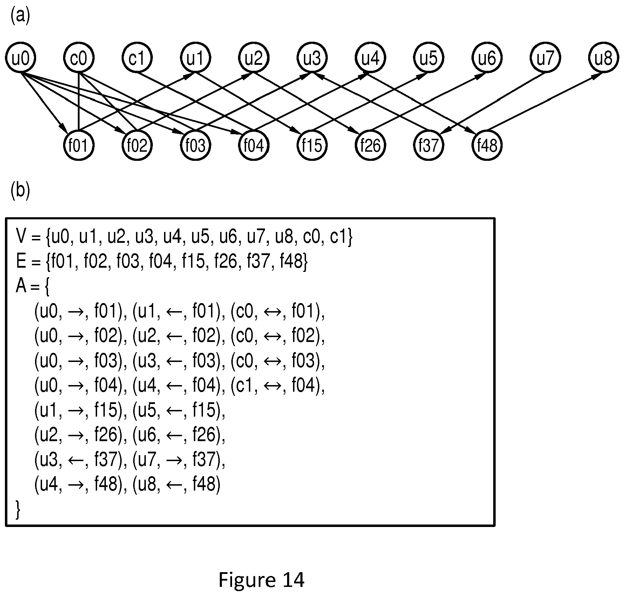

In step S114 of Figure 12, Figure 13b is converted to the bipartite graph *(V, E, A)* in Figure 14b. Figure 14a is a visually understandable version (not necessary for the machine processing in embodiments).

First, *V* is created from $\overline{V}$ by making V identical to $\overline{V}$. Next, E is created from all the first elements of the entries in $\overline{E}$. Then, A is created by generating one or more edges from each entry in E.

For example, from *(f01, {u0}, {u1}, {c0})* in Figure 13a, the three edges *(u0, →, f01), (u1, ←, f01),* and *(c0, ↔, f01)* are created in the following way.

For each member in the second element of *(f01, {u0}, {u1}, {c0})*, an edge made from the member, "→", and the first element of *(f01, {u0}, {u1}, {c0})* is created. Since the second element has only *u0* and the first element is *f01*, *(u0, →, f01)* is created.

For each member in the third element of *(f01, {u0}, {u1}, {c0})*, an edge made from the member, "←", and the first element of *(f01, {u0}, {u1}, {c0})* is created. Since the third element has only *u1* and the first element is *f01*, *(u1, ←, f01)* is created.

For each member in the fourth element of *(f01, {u0}, {u1}, {c0})*, an edge made from the member, "↔", and the first element of *(f01, {u0}, {u1}, {c0})* is created. Since the fourth element has only *c0* and the first element is *f01*, *(c0, ↔, f01)* is created.

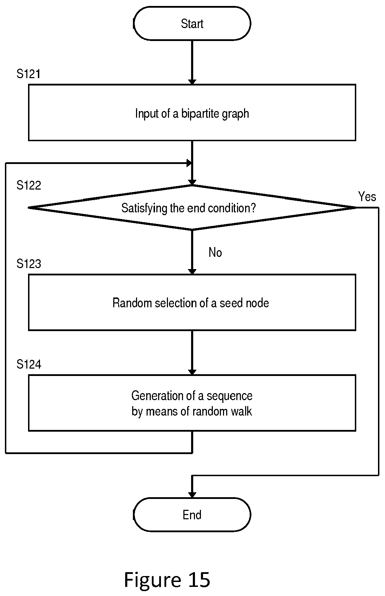

The next stage is graph sampling, as show in Figure 15.

In step S121 a bipartite graph is inputted. Here we assume that the graph in Figure 14 is inputted. Until a predefined end condition is satisfied, the loop of Step S122, Step S123, and Step 124 is repeated. Step S122 checks an end condition is satisfied. The end condition may be the number of generated sequences. Here it is assumed that the number is 168. Step S123 and step S124 may contain randomness and are affected by fluctuation in general, but here it is assumed that each candidate of a random selection is selected by its exact probability.

In Step 123 a seed node is selected. Here it is assumed that it is selected from the entries in $V$. It may be selected from the entries in $V$ and $E$. It is also assumed that a node with no outgoing edge $(\rightarrow$ or $\leftrightarrow)$ is not selected.

Since each of $u3$, $u5$, $u6$ and $u8$ in $V$ has no outgoing edge, the other 7 nodes $(u0, u1, u2, u4, u7, c0, c1)$ in $V$ are the candidates. Each node is selected 24 (= 168/7) times.

In step S124 of Figure 15, a random walk is performed. A random walk may be stopped when an arbitrary condition is satisfied. It is assumed that it is stopped when the length reaches 3 or no next candidate is found. It is also assumed that $\lambda_w(.) = 1$. So the formula of $p(e_x|v_x)$ is simplified to $p(e_x|v_x) = 1/|E_o(v_x)|$.

Start from $u0$, i.e. $v_1 = u0$. $E_o(u0)$ is a set of edge nodes satisfying $(u0, \rightarrow, e)$ or $(u0, \leftrightarrow, e)$ in $A$. Since $(u0, \rightarrow, f01)$, $(u0, \rightarrow, f02)$, $(u0, \rightarrow, f03)$, and $(u0, \rightarrow, f04)$ exist in A, $E_o(u0) = \{f01, f02, f03, f04\}$ and $pf01|u0) = p(f02|u0) = p(f03|u0) = p(f04|u0) = 1/4$.

Select $f03$ as $e_1$ from $E_o(u0)$, i.e. $e_1 = f03$. $V_o(f03)$ is a set of vertex nodes satisfying $(v, \leftarrow, f03)$ or $(v, \leftrightarrow, f03)$ in $A$. Since $(u3, \leftarrow, J03)$ and $(c0, \leftrightarrow, f03)$ exist in A, $V_o(f03) = \{u3, c0\}$ and $V_o(f03)\backslash\{u0\} = \{u3, c0\}$. So, $p(u3|f03) = p(c0|f03) = 1/2$.

The probability of the first transition is calculated as follows.

When $u3$ is selected as $u2$, $p[(u0, f03, u3)] = p(f03|u0) * p(u3|f03) = (1/4) * (1/2) = 1/8$.

When $c0$ is selected as $u2$, $p[(u0, f03, c0)] = pf03|u0) * p(c0|f03) = (1/4) * (1/2) = 1/8$.

Next, the second transitions are explained.

(1) the first transition is $(u0, f03, u3)$, that is, $v_2 = u3$. $E_o(u3)$ is a set of edge nodes satisfying $(u3, \rightarrow, e)$ or $(u3, \leftrightarrow, e)$ in $A$. Since there is no such $e$ in $A$, $E_o(u3)$ is empty. In this case, the random walk stops here because no next candidate is found. So the path $(u0, f03, u3)$ is generated 24 * $p[(u0, f03, c0)] = 24 * (1/8) = 3$ times.

(2) the first transition is $(u0, f03, c0)$, that is, $v_2 = c0$. $E_o(c0)$ is a set of edge nodes satisfying $(u3, \rightarrow, e)$ or $(u3, \leftrightarrow, e)$ in $A$. Since $(c0, \leftrightarrow, f01)$, $(c0, \leftrightarrow, f02)$, and $(c0, \leftrightarrow, f03)$ exist in A, $E_o(c0) = \{f01, f02, f03\}$ and $p(f01|c0) = p(f02|c0) = p(f03|c0) = 1/3$.

Select $f01$ as $e_2$ from $E_o(c0)$, i.e. $e_1 = f01$.

$V_o(f01)$ is a set of vertex nodes satisfying $(v, \leftarrow, f01)$ or $(v, \leftrightarrow, f01)$ in $A$. Since $(u1, \leftarrow, f01)$ and $(c0, \leftrightarrow, f01)$ exist in A, $V_o(f01) = \{u1, c0\}$ and $V_o(f01)\backslash\{c0\} = \{u1\}$. **So**, $p\{u1\} = 1$. Note that $u1$ is not a candidate of $v_2$.

The probability of the second transition is calculated as follows:

Only $u1$ is selected as $v_3$, $p[(c0, f01, u1)] = p(f01|c0) * p(u3|f03) = (1/3) * 1 = 1/3$.

So the path $(u0, f03, c0, f01, u1)$ is generated 24 * $p[(u0, f03, c0)] * p[(c0, f01, u1)] = 24 * (1/8) * (1/3) = 1$ time.

In a similar way, other paths are generated. After the loop repeats 168 times (the predefined number of times), the process exits to Yes in Step 122.

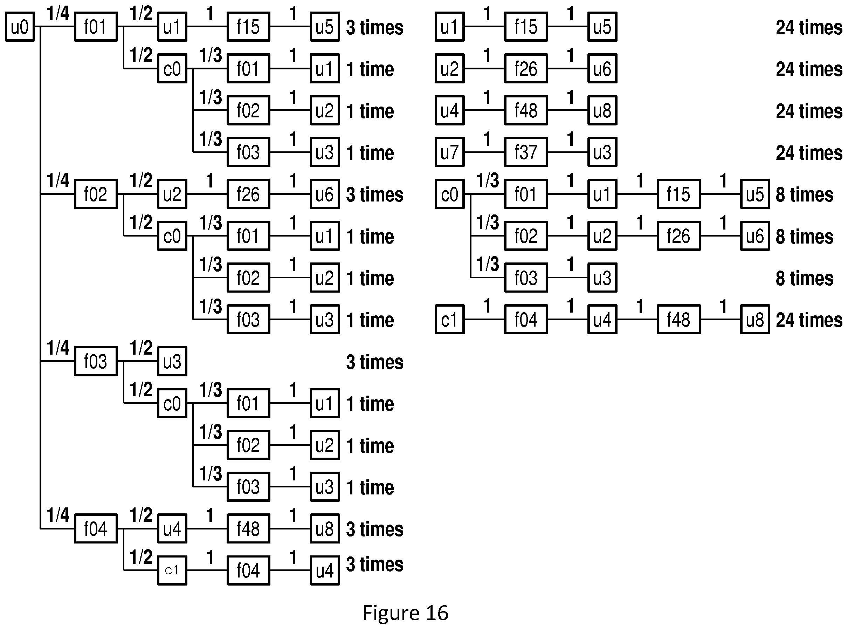

Figure 16 is a summary of the generated paths. A rectangle denotes a vertex node or an edge node. The number attached to a line connecting two rectangles denotes a probability $p(e_x|v_x)$ or $p(v_{x+1}|e_x)$. A series of connected rectangles from left to right corresponds to a path. The number attached to a path denotes how many times the path is generated. It is calculated by 168 * all the probabilities on the path. For example, the first line shows that $(u0, f01, u1, f15, u5)$ is generated 3 times. The second line shows that $(u0, f01, c0, f01, u1)$ is generated 1 time.

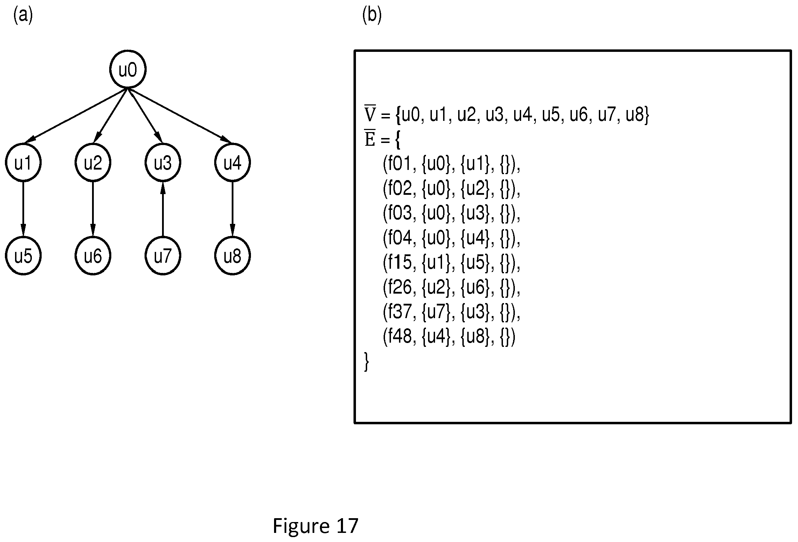

In the prior art, a hyperedge connecting more than 2 nodes cannot be effectively processed. With normal graph representation, Figure 13a can be represented as Figure 17a by ignoring $c0$ and $c1$. For this graph various random walk techniques such as (Grover and Leskovec - 2016) and (Ristoski and Paulheim - 2016) can be applied. In invention embodiments such a method may be imitated as follows.

Working Example Part 1: Comparative Effects

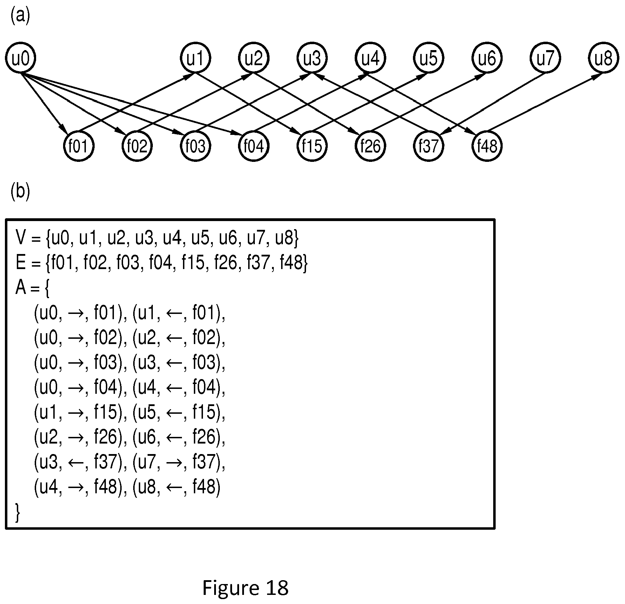

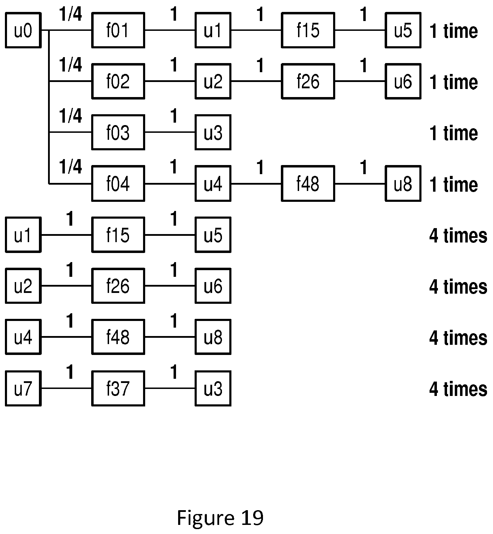

First, Figure 17a can be represented as Figure 17b as in Figure 13. The bipartite converter converts Figure 17 to Figure 18 in a similar way as it converts Figure 13 to Figure 14. With Figure 18 the graph sampler generates sequences shown in Figure 19 in a similar way as it generates sequences shown in Figure 16, with a number of generated sequences of 20.

As is obvious because Figure 18 does not contain $c0$ or $c1$, paths containing $c0$ or $c1$ are never generated as shown in Figure 19. This means that invention embodiments are superior because they generate paths containing $c0$ and $c1$.

A method ignoring directionality such as "On the Representation and Embedding of Knowledge Bases Beyond Binary Relations", 2013 (Wen et al. - 2016) generates undesirable paths. In invention embodiments such a method may also be imitated as follows.

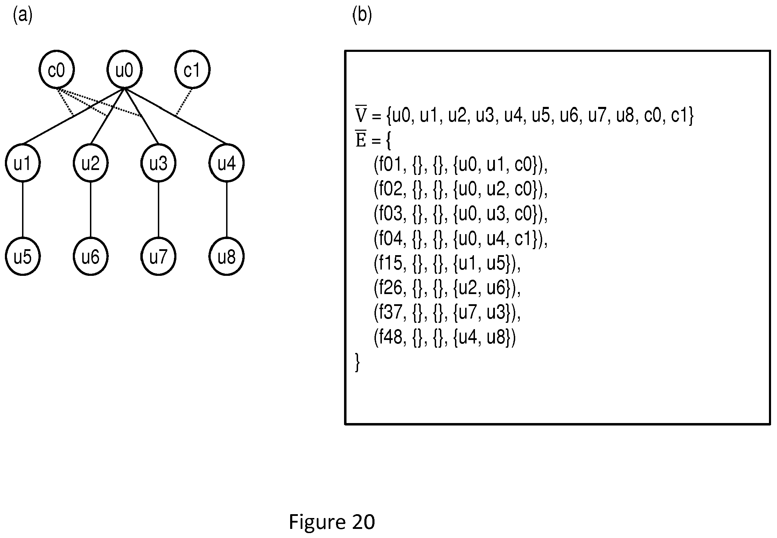

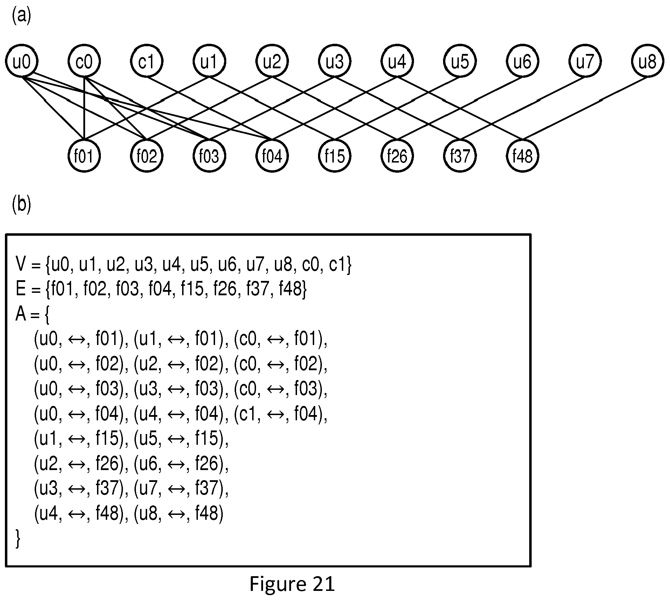

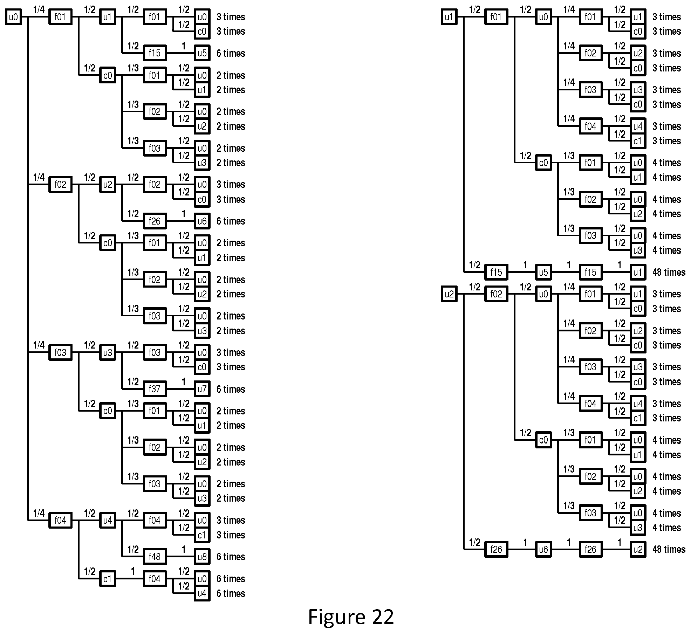

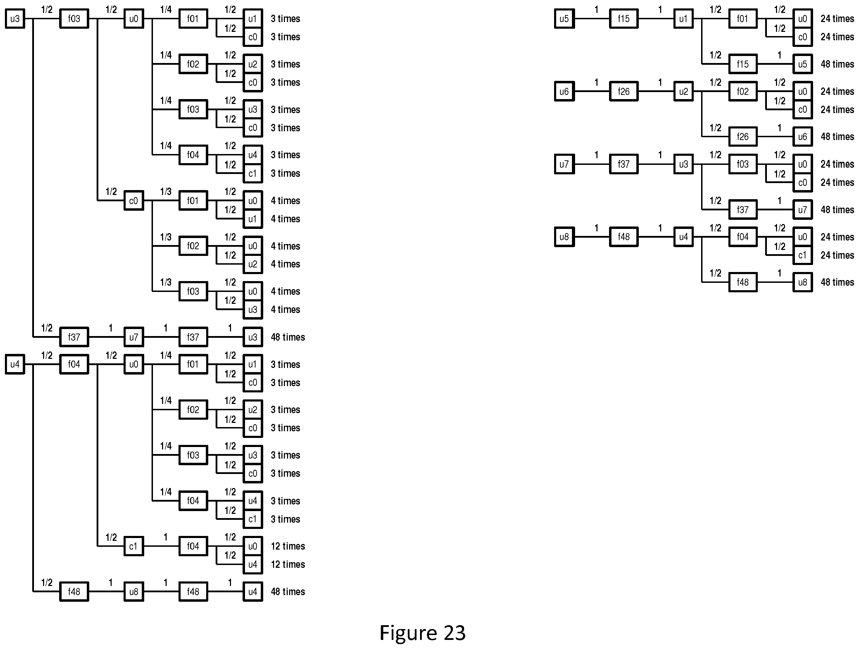

When ignoring directionality, Figure 13 becomes Figure 20. The bipartite converter converts Figure 20 to Figure 21 in a similar way as it converts Figure 13 to Figure 14. With Figure 21, the graph sampler generates sequences shown in Figure 22, Figure 23, and Figure 24 in a similar way as it generates sequences shown in Figure 16, with a supposed number of generated sequences of 1056.

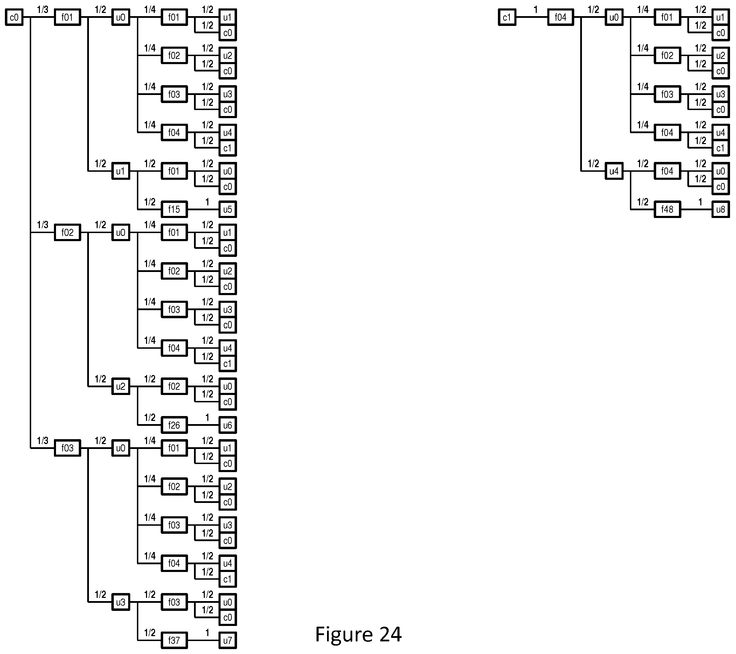

In Figure 24, the sequence (*c0, f03, u3*) further continues and (*c0, f03, u3, f03, u0*), (*c0, f03, u03, f03, c0*), and (*c0, f03, u3, f37, u7*) are generated. In the meantime, in Figure 16, the sequence (*c0, f03, u3*) stops at *u3* and only (*c0, f03, u3*) is generated. This shows that the invention embodiments are superior because they take directionality into account and prevent generation of undesirable paths.

The Second Worked Example considers the addition of the distributional analysis, as shown in step S103 of Figure 9.

The input to the distributional analyser is Figure 16 for invention embodiments, Figure 19 for the normal graph version, and Figure 22, Figure 23 and Figure 24 for the no-directionality version, as explained above.

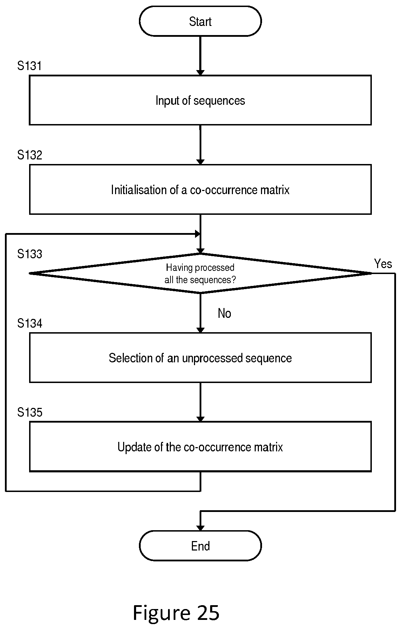

The process taking place in the distributional analyser is shown in Figure 25.

In step S131 a list of sequences is inputted. Here the sequence in Figure 16 is inputted. The number of times a sequence in the list is generated is the number denoted on the right side. For example, the list contains 3 sequences of (*u0, f01, u1, f15, u5*).

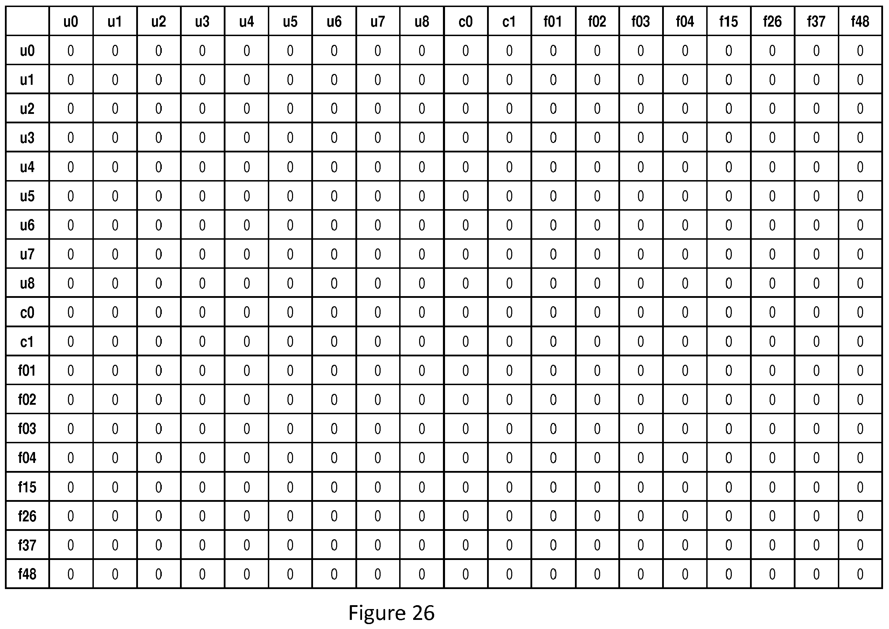

In step S132 a co-occurrence matrix is initialized (fully zeroed) as shown in Figure 26.

Until all the sequences in Figure 16 are processed (as questioned in step S133), the loop of step S133, step S134, and step S135 is repeated.

In step S135, the matrix is updated, for example according to the formulas in the section entitled "Distributional Vectorization". For simplicity, a less complex variation is shown below:

For each possible pair $(s_i, s_j)$ consisting of two elements in the sequence (note: excluding a pair where

$$s_i = s_j), F(s_i, s_j) := F(s_i, s_j)+1 \text{ and } F(s_j, s_i) := F(s_j, s_i)+1$$

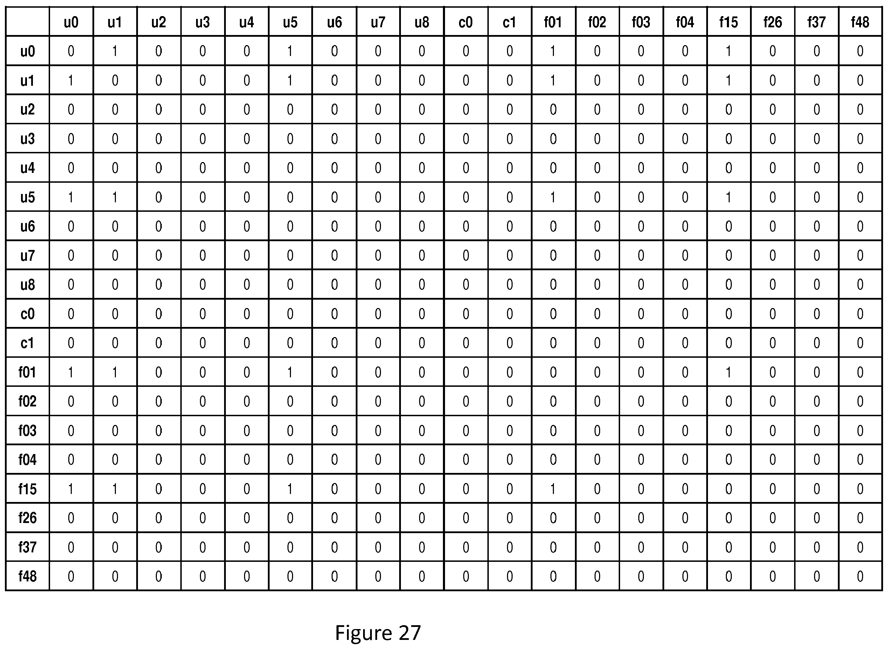

(1st iteration) In Step 134, suppose the first sequence (*u0, f01, u1, f15, u5*) of the three is selected. In Step 135, since the possible pairs are (*u0, f01*), (*u0, u1*), (*u0, f15*), (*u0, u5*), (*f01, u1*), (*f01, f15*), (*f01, u5*), (*u1, f15*), (*u1, u5*), and (*f15, u5*), the cell in *F* corresponding to each pair is incremented by 1, and *F* is updated to Figure 27 (updated elements are shaded).

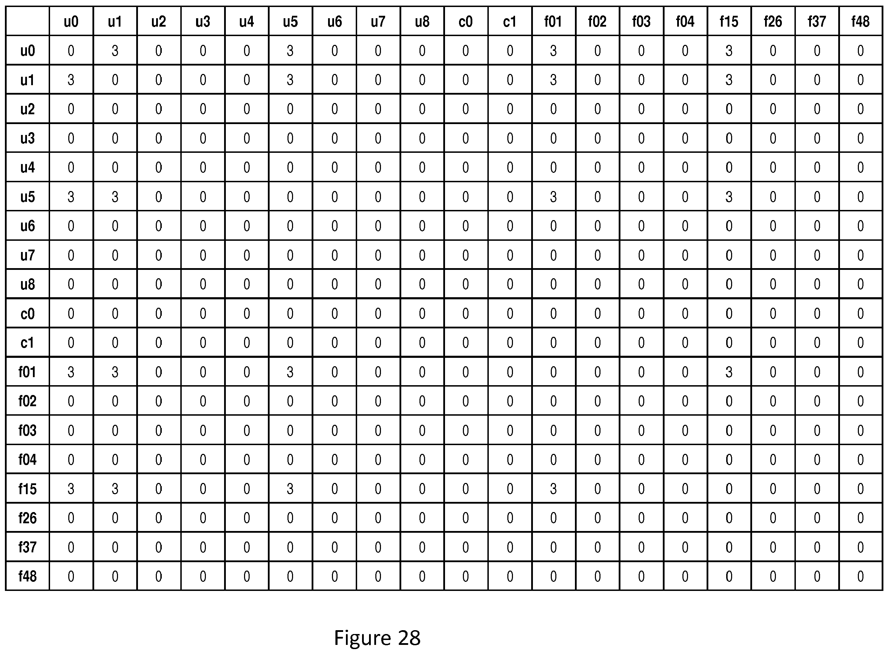

(2nd and 3rd iterations) Suppose the second and the third sequences (*u0, f01, u1, f15, u5*) of the three are selected. After the 3rd iteration, *F* is updated to Figure 28 by similar calculations.

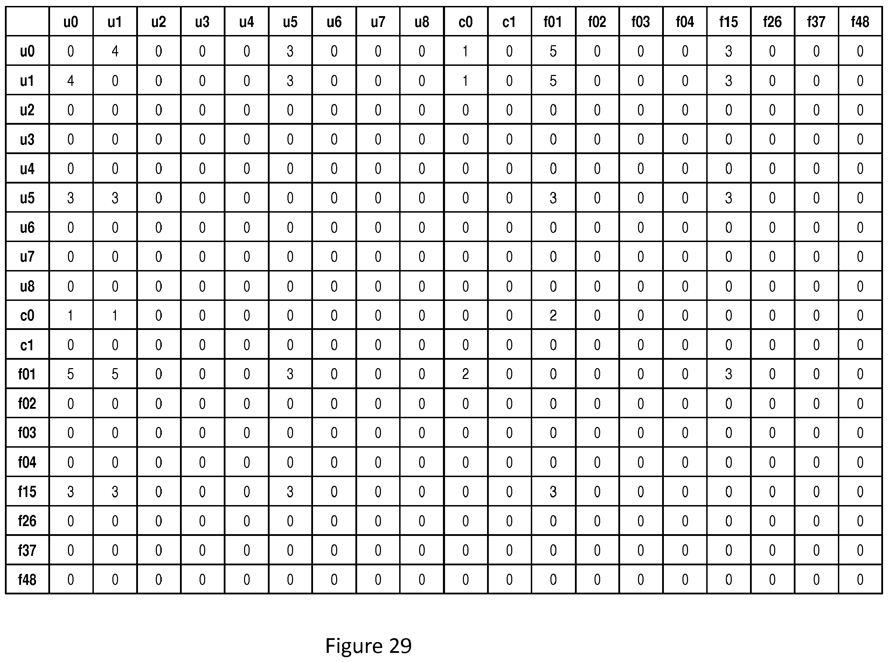

(4th iteration) In Step 134, suppose (*u0, f01, c0, f01, u1*) is selected. In Step 135, since the possible pairs are (*u0, f01*), (*u0, c0*), (*u0, f01*), (*u0, u1*), (*f01, c0*), (*f01, u1*), (*c0, f01*), (*c0, u1*), and (*f01, u1*) (note: (*f01, 101*) is excluded), the cell in *F* corresponding to each pair is incremented by 1, and *F* is updated to Figure 29.

All the other sequences in Figure 16 are processed in a similar way.

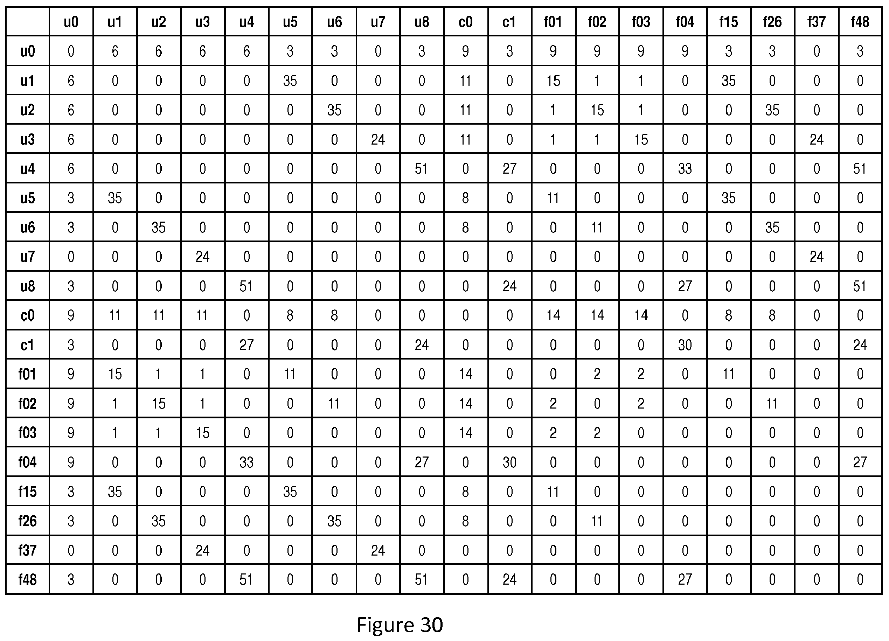

Finally, the co-occurrence matrix becomes Figure 30.

Working Example Part 2: Comparative Effects

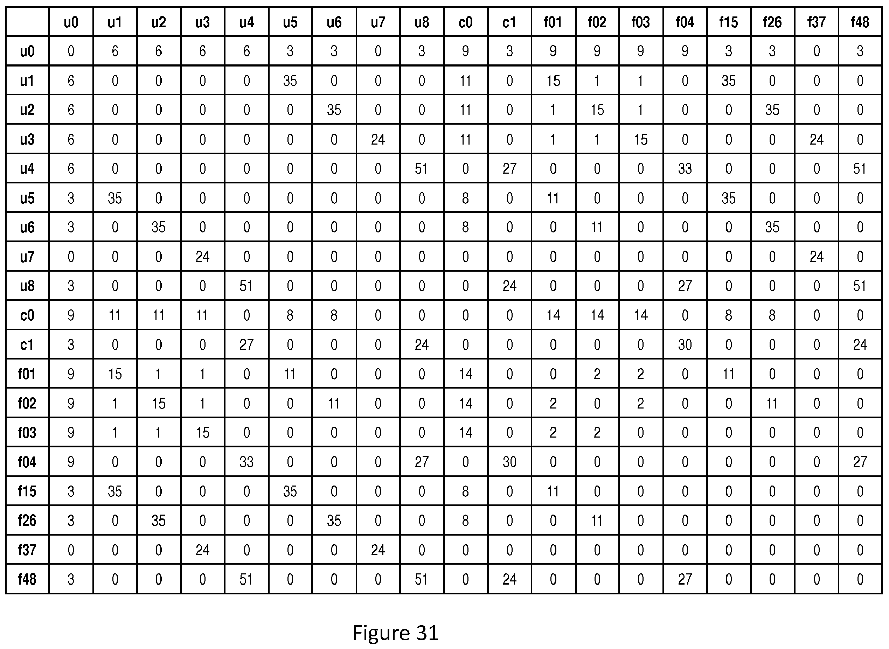

For a comparison with a method with normal graph representation, the distributional analyser is applied to Figure 19. In a similar way to the one for generating Figure 30 from Figure 16, the co-occurrence matrix in Figure 31 is obtained.

Invention embodiments (Figure 30):

The embedding of *u5* is a vector corresponding to the *u5* row in Figure 30. That is,

$$u5 = (3, 35, 0, 0, 0, 0, 0, 0, 0, 8, 0, 11, 0, 0, 0, 35, 0, 0, 0)$$

Similarly,

$$u6 = (3, 0, 35, 0, 0, 0, 0, 0, 0, 8, 0, 0, 11, 0, 0, 0, 35, 0, 0)$$

$$u8 = (3, 0, 0, 0, 51, 0, 0, 0, 0, 0, 24, 0, 0, 0, 27, 0, 0, 0, 51)$$

Calculate the cosine similarity of *u5* and *u6,* and of *u5* and *u8*.

$$\text{sim(u5, u6)} = \frac{u5 \cdot u6}{|u5||u6|} = 0.0276$$

$$\text{sim(u5, u8)} = \frac{u5 \cdot u8}{|u5||u8|} = 0.0022$$

A method with normal graph representation (Figure 31):
Similarly,

$$u5 \quad = \quad (1, \quad 5, \quad 0, \quad 0, \quad 0, \quad 0, \quad 0, \quad 0, \quad 0, \quad 1, \quad 0, \quad 0, \quad 0, \quad 5, \quad 0, \quad 0, \quad 0)$$

$$u6 \quad = \quad (1, \quad 0, \quad 5, \quad 0, \quad 0, \quad 0, \quad 0, \quad 0, \quad 0, \quad 0, \quad 1, \quad 0, \quad 0, \quad 0, \quad 5, \quad 0, \quad 0)$$

$$u8 = (1, 0, 0, 0, 5, 0, 0, 0, 0, 0, 0, 0, 1, 0, 0, 0, 5)$$

and

$$\text{sim(u5, u6)} = \frac{u5 \cdot u6}{|u5||u6|} = 0.0192$$

$$\text{sim(u5, u8)} = \frac{u5 \cdot u8}{|u5||u8|} = 0.0192$$

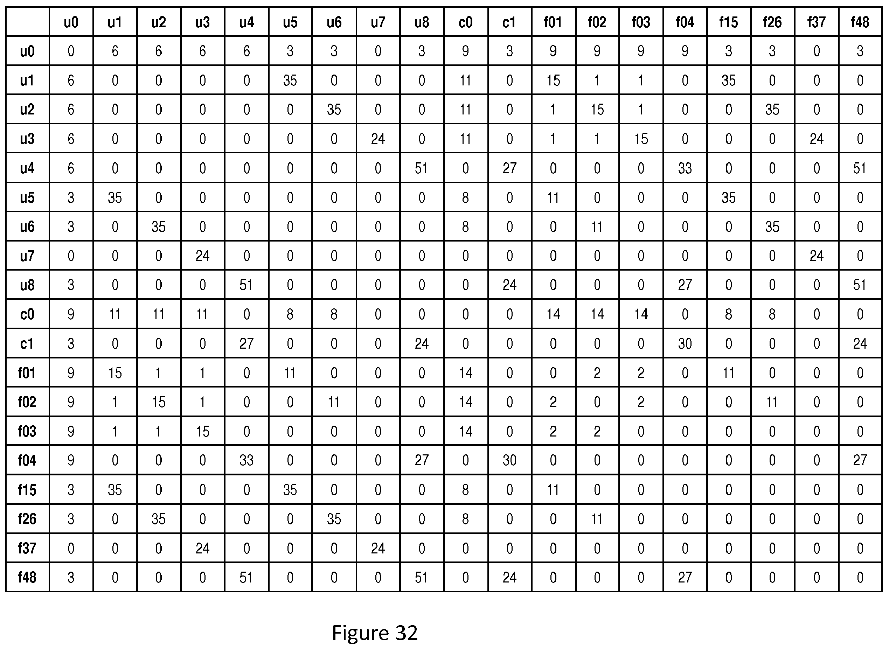

With invention embodiments *u5* is more similar to *u6* than to *u8,* while with the other method *u5* is equally similar to *u6* and *u8*. In Figure 13, *u5, u6,* and *u8* have a similar structure *"u0 → (another node) → (the node concerned)"* but the first edge is connected to *c0* for *u5* and *u6* while it is connected to *c1* for *u8.* Therefore it is natural to think that *u5* is more similar to *u6* than to *u8,* which invention embodiments demonstrate, while the other method does not.
Compare the generated sequences in Figure 15 and Figure 19. In Figure 19 the sequences are symmetrical with regard to *u5, u6,* and *u8*. But in Figure 15, for example, when looking at the sequences starting from *c0* and *c1, c0* appears in the sequence containing *u5* and the one containing *u6* while *c1* appears in the sequence containing *c8*. This implies that *u5* is more similar to *u6* than to *u7*.
This observation is the effect of treating hyperedges appropriately.
For comparison with a method ignoring directionality, the distributional analyser is applied to Figure 22, Figure 23 and Figure 24. In a similar way to the one for generating Figure 30 from Figure 15, the co-occurrence matrix in Figure 32 is obtained.
Invention embodiments (Figure 30):
The embedding of *u5* is a vector corresponding to the *u5* row in Fig. D5. That is,

$$u5 = (3, 35, 0, 0, 0, 0, 0, 0, 0, 8, 0, 11, 0, 0, 0, 35, 0, 0, 0)$$

Similarly,

$$u6 \quad = \quad (3, \quad 0, \quad 35, \quad 0, \quad 0, \quad 0, \quad 0, \quad 0, \quad 0, \quad 8, \quad 0, \quad 0, \quad 11, \quad 0, \quad 0, \quad 0, \quad 35, \quad 0, \quad 0)$$

$$u7 = (0, 0, 0, 24, 0, 0, 0, 0, 0, 0, 0, 0, 0, 0, 0, 0, 0, 24, 0)$$

Calculate the cosine similarity of *u5* and *u6,* and of *u5* and *u8*.

$$sim(u5,u6) = \frac{u5 \cdot u6}{|u5||u6|} = 0.0276$$

$$sim(u5, u7) = \frac{u5 \cdot u7}{|u5||u7|} = 0$$

A method with normal graph representation (Fig. F1):
Similarly,

$$u5 = (30, \quad 254, \quad 0, \quad 0, \quad 0, \quad 0, \quad 0, \quad 0, \quad 0, \quad 32, \quad 0, \quad 62, \quad 0, \quad 0, \quad 0, \quad 350, \quad 0, \quad 0, \quad 0)$$

$$u6 = (30, \quad 0, \quad 254, \quad 0, \quad 0, \quad 0, \quad 0, \quad 0, \quad 0, \quad 32, \quad 0, \quad 0, \quad 62, \quad 0, \quad 0, \quad 0, \quad 350, \quad 0, \quad 0)$$

$$u7 = (30, 0, 0, 254, 0, 0, 0, 0, 0, 32, 0, 0, 0, 62, 0, 0, 0, 350, 0)$$

and

$$sim(u5, u6) = \frac{u5 \cdot u6}{|u5||u6|} = 0.0100$$

$$sim(u5, u7) = \frac{u5 \cdot u7}{|u5||u7|} = 0.0100$$

With invention embodiments *u5* is more similar to *u6* than to *u7,* while with the other method *u5* is equally similar to *u6* and *u7*. In Figure 13, *u5, u6,* and *u7* have a similar structure *"u0 -> (another node) -?- (the node concerned)"* but the second edge is a forward edge for *u5* and *u6* while it is a backward edge for *u7*. Therefore it is natural to think that *u5* is more similar to *u6* than to *u7,* which our invention achieves while the other method does not.
Try to compare the generated sequences in Figure 15 and Figure 22 to Figure 24. In Figure 22 to Figure 24 the sequences are symmetric with regard to *u5, u6,* and *u7*. But in Figure 15, for example, when looking at the sequences starting from *c0, u5* appears in a sequence starting from *c0* and *u6* appears in another sequence starting from *c0,* while *u7* does not appear in any sequence starting from *c0*. This implies that *u5* is more similar to *u6* than to *u7*. This observation is the effect of treating directionality appropriately.
A concrete instantiation in the domain as follows:
In biology, there is the MST/Hippo Pathway responsible for cell apoptosis (cell death) and which as usual may also cause the antagonist action cell survival and proliferation. For that example, the actual instantiation of each entity and context in the combined graph (shown for example in Figure 13) may be as follows:

    C0: Pro-apoptotic
    C1: Anti-apoptotic
    U0: LATS1/2
    U1: TAZ
    U2: FOXO
    U3:BAX
    U4:YAP
    U5 : P73
    U6 : AIF1
    U7:PUMA
    U8:TEAD

All the relations (fxx) are phosphorylations and point from a kinase to a substrate.

The effect of the Working Example: Part 2 is the following: using context information (pro/anti-apoptotic) in invention embodiments allow predictions that $u5$ (P73) is closer to $u6$ (AIF1) than to $u8$ (TEAD). Indeed both AIF1 and P73 are part of the pro-apoptotic part of the pathway whereas TEAD is involved in the anti-apoptotic. This is very important in cancer research as usually it is advantageous for cancer cells not to proliferate but on the contrary for those cells to die.

Also, based on the connections of the proteins, the biological function of some proteins may be inferred ((Context C0 and C1 pro/anti-apoptotic may be viewed as biological functions). In particular from this small example we may see that TEAD protein (U8) is more likely related to anti-apoptotic (closer to C1 than to C0). This information is crucial for biology experimentalists as it gives them an indication (which saves time) of which experiment to perform for the development of new drug targets.

A GUI (Graphical User Interface) may be provided for the user to select knowledge graphs to be combined using the methods described herein and to enter data such as proteins of interest to allow protein properties to be derived, for example from similarity of two proteins, and displayed to the user. For example, one window may display a field to enter or select a protein of interest and the same or another window may allow display and/or selection of knowledge graphs for input. Again, the same or another window may display results, such as protein similarity etc.

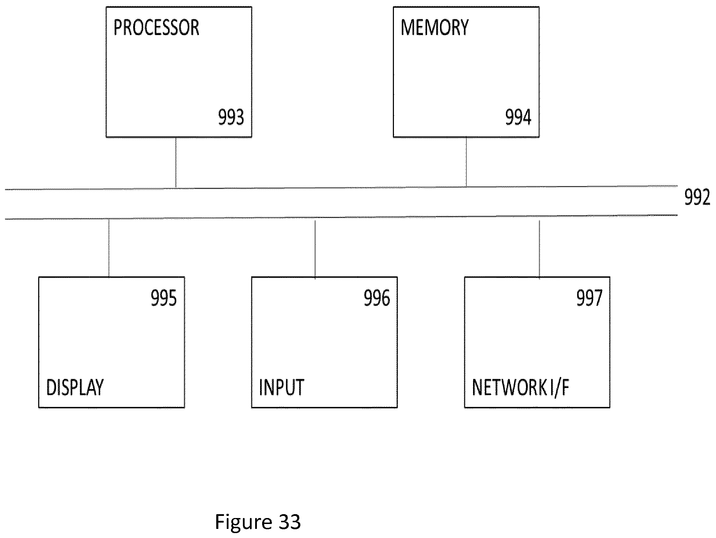

Figure 33 is a block diagram of a computing device, such as a data storage server, which embodies the present invention, and which may be used to implement a method inferring a property of a protein. The computing device comprises a processor 993, and memory, 994. Optionally, the computing device also includes a network interface 997 for communication with other computing devices, for example with other computing devices of invention embodiments.

For example, an embodiment may be composed of a network of such computing devices. Optionally, the computing device also includes one or more input mechanisms such as keyboard and mouse 996, and a display unit such as one or more monitors 995. The components are connectable to one another via a bus 992.

The memory 994 may include a computer readable medium, which term may refer to a single medium or multiple media (e.g., a centralized or distributed database and/or associated caches and servers) configured to carry computer-executable instructions or have data structures stored thereon. Computer-executable instructions may include, for example, instructions and data accessible by and causing a general purpose computer, special purpose computer, or special purpose processing device (e.g., one or more processors) to perform one or more functions or operations. Thus, the term "computer-readable storage medium" may also include any medium that is capable of storing, encoding or carrying a set of instructions for execution by the machine and that cause the machine to perform any one or more of the methods of the present disclosure. The term "computer-readable storage medium" may accordingly be taken to include, but not be limited to, solid-state memories, optical media and magnetic media. By way of example, and not limitation, such computer-readable media may include non-transitory computer-readable storage media, including Random Access Memory (RAM), Read-Only Memory (ROM), Electrically Erasable Programmable Read-Only Memory (EEPROM), Compact Disc Read-Only Memory (CD-ROM) or other optical disk storage, magnetic disk storage or other magnetic storage devices, flash memory devices (e.g., solid state memory devices).

The processor 993 is configured to control the computing device and execute processing operations, for example executing code stored in the memory to implement the various different functions of the bipartite converter, graph sampler and distributional analyser described here and in the claims. The memory 994 stores data being read and written by the processor 993. As referred to herein, a processor may include one or more general-purpose processing devices such as a microprocessor, central processing unit, or the like. The processor may include a complex instruction set computing (CISC) microprocessor, reduced instruction set computing (RISC) microprocessor, very long instruction word (VLIW) microprocessor, or a processor implementing other instruction sets or processors implementing a combination of instruction sets. The processor may also include one or more special-purpose processing devices such as an application specific integrated circuit (ASIC), a field programmable gate array (FPGA), a digital signal processor (DSP), network processor, or the like. In one or more embodiments, a processor is configured to execute instructions for performing the operations and steps discussed herein.

The display unit 997 may display a representation of data stored by the computing device and may also display a cursor and dialog boxes and screens enabling interaction between a user and the programs and data stored on the computing device. The input mechanisms 996 may enable a user to input data and instructions to the computing device. For example the user may instruct the computing device which graphs to input and combine and which property to compare based on the completed feature matrix.

The network interface (network I/F) 997 may be connected to a network, such as the Internet, and is connectable to other such computing devices via the network. For example, the input may be of knowledge graphs from the network. The network I/F 997 may control data input/output from/to other apparatus via the network. Other peripheral devices such as microphone, speakers, printer, power supply unit, fan, case, scanner, trackerball etc may be included in the computing device.

The bipartite converter may comprise processing instructions stored on a portion of the memory 994, the processor 993 to execute the processing instructions, and a portion of the memory 994 to store the graph during the execution of the processing instructions.

The output of the bipartite converter may be stored on the memory 994 and/or on a connected storage unit, such as bipartite graph DB 50.

The graph sampler may comprise processing instructions stored on a portion of the memory 994, the processor 993 to execute the processing instructions, and a portion of the memory 994 to store sequences during the execution of the processing instructions. The output of the graph sampler may be stored on the memory 994 and/or on a connected storage unit, such as sequence DB 60.

The distributional analyser may comprise processing instructions stored on a portion of the memory 994, the processor 993 to execute the processing instructions, and a portion of the memory 994 to store the feature matrix and cosine similarity during the execution of the processing instructions. The output of the distributional analyser may be stored on the memory 994 and/or on a connected storage unit, such as co-occurrence matrix DB 70.

Methods embodying the present invention may be carried out on a computing device such as that illustrated in Figure 33. Such a computing device need not have every component illustrated in Figure 33, and may be composed of a subset of those components. A method embodying the present invention may be carried out by a single computing device in communication with one or more data storage servers via a network. The computing device may be a data storage itself storing the graphs/feature matrix.

A method embodying the present invention may be carried out by a plurality of computing devices operating in cooperation with one another. One or more of the plurality of computing devices may be a data storage server storing at least a portion of a graph or matrix.

**Scope and Limitations**

The main scope of the system is generation of actionable and efficient representations of graph structures, with a specific focus on knowledge graphs that encode relationships of different arities and directions such as bio-informatic context-dependent pathway networks or binary statements extracted from publications and their provenance such as bio-informatic context-dependent pathway networks or binary statements extracted from publications and their provenance.

The main limitation is the overhead caused by the underlying bipartite representation. While this allows for universal representation of arbitrary graph structures, representations of standard binary graphs or knowledge graphs composed of triples will be inherently less efficient than representations using standard graphs and related data structures.

**Functionality, Effectiveness**

Embodiments of the invention will be especially beneficial in use cases where non-uniform knowledge graphs have to be processed together (for instance, analysis of binary relations in protein interaction networks together with context-dependent n-ary relations in pathway databases). The unprecedented ability to handle diverse data sets together may lead to automated discoveries that would otherwise be principally impossible.

Systems biology cancer research is an example where the invention may have a particularly positive effect: one aspect of systems biology work involves the modelling of signalling pathways (set of protein interactions happening in the context of one or multiple pathways that together explain a cancer). Being able to discover missing protein interactions is therefore very important. With invention embodiments, a system may make such prediction based on protein embeddings computed from not only binary relations of protein interactions, but as well context information of pathways, and taking into account provenance scientific article of two entities co-occurring. As demonstrated in the embodiment examples, being able to handle mixed arity and mixed directionality allow a system to take advantage of richer information and consequently build a more accurate embedding.

The flexibility of the approach will also lead to shorter computational experimentation times. Embodiments of the invention allow for computing distributional representations from graph structures of any type and directionality (including mixed arity and directionality relations). The distributional representation is an embedding on its own, and may also be straightforwardly used for computing other, lower-rank embeddings.

Thus embodiments have the following practical effects of enabling efficient representation of and inference from graph structures with mixed relation arities and directionality. No prior art can handle such structures at the moment even though there are important use cases that would benefit from that.

Embodiments may provide any of the following advantageous features:

A system/method that may take any graph structure (standard graph, multigraph, hypergraph) with potentially mixed directionality of edges and transform it into a list of node and edge sequences and/or feature matrices that distinguish between the entity and relation semantics and may be consequently used for computing graph structure embeddings or used as embeddings themselves.

A system/method as above where the transformation utilises a universal representation of graph structures based on bipartite graphs.

A system/method as above where the underlying universal bi-partite graph representation utilises integer encoding of nodes and edges for memory efficiency and boolean sparse matrices for fast graph operations.

A system/method as above where the transformation method utilises traversal-based sampling (e.g., depth-first, breadth-first, best-fist search or random walks) in order to generate the node and edge sequences.

A system/method as above where the feature matrices are produced from the node and edge sequences using distributional principles (i.e. co-occurrence analysis).

A system/method as above where feature selection based on frequency characteristics of the elements occurring in the node and edge sequences may be applied when constructing the feature matrices.

**Glossary**

**Arity** A property of a graph indicating the maximum number of nodes connected by an edge.

**Knowledge Graph** A collection of items (represented by *nodes/vertices*) any of which may be connected by links (*such* as typed binary relations) between them. They can be understood as a graph-structured knowledge base. A generalisation to structure connecting more than two nodes is possible and required in some applications.

**Embedding** A mapping of elements of one structure (*e.g.* a node or relation in a graph, or a phrase in a set of natural language sentences) to vectors in a (typically low-rank) continuous vector space. Used primarily for convenient (linear) representation of structured information so that some interesting patterns can be discovered in it.

**Graph** A mathematical structure representing relationships (edges) between pairs of entities (nodes or vertices). Directed or undirected graphs are typically distinguished (in directed graphs, an edge from node *A* to node *B* means something different than an edge from node *B* to node *A*, while in undirected graphs it means the same). Typically, no edge or node labels are considered. Also, multiple connections between the same two nodes (multi-edges) and loops (edges "connecting" a node to itself) are forbidden in standard graphs.

**Labelled Graph** A generalisation of graph that can associate labels with nodes and/or edges.

**Weighted Graph** A generalisation of graph that can associate weights with its edges to reflect their relative importance.

**Multigraph** A generalisation of graph that can contain loops and multi-edges (e.g., two edges between the same two vertices/nodes).

**Hypergraph** A generalisation of graph that can contain hyperedges that link more than two nodes.

**Bipartite Graph** A graph that has to distinct set of nodes *U, V* such that there are no edges connecting nodes within *U* or V. In otherwords, only edges connecting nodes from *U* with nodes from V are allowed.

**Graph Structure** Any type of graph or its generalisation (*e.g.* multigraph or hypergraph).

**Graph Path** A sequence $(u_1, e_1, u_2, e_2, .., e_{k-1}, u_k)$ of nodes $u_x$ and edges $e_y$ of length *2k - 1* in a graph structure $G$, where $k$ is a number of nodes in the path and for any $u_i, e_i, u_{i+1}$ in the path, $e_i$ encodes the edge between $u_i$ and $u_i+1$. The number of nodes occurring in the path can possibly be infinite.

**Graph Traversal** A graph path where each transition between the nodes is chosen using a specific principle.

**Depth-First Search** A type of graph traversal. A graph path that attempts for an exhaustive search of the graph (*i.e.,* visiting all nodes). Each transition between the nodes attempts to go deeper into the graph; if no new nodes can be visited, the traversal backtracks to the last unvisited node.

**Breadth-First Search** A type of graph traversal. A graph path that attempts for an exhaustive search of the graph (*i.e.,* visiting all nodes). Each transition between the nodes attempts to exhaustively visit all neighbours of the previous node before going deeper; if no new nodes can be visited, the traversal backtracks to the last unvisited node.

**Best-First Search** A type of graph traversal. A graph path where each transition between the nodes is chosen based on an application-dependent heuristics that determines the "promise" of the next node candidate for most efficient search.

**Random Walk** A type of graph traversal. A graph path where each transition between the nodes is chosen randomly. The choice may either be uniform among the available edges, or based on the edge weights in weighted graph structures.

**Distributional Representation** Representation of elements in a structure (*e.g.* nodes or relations in a graph, or phrases in a text corpus) based on other elements it frequently co-occurs with it. Typically encoded using continuous vectors that reflect particular co-occurrence scores w.r.t. other elements in the data.

**Claims**

1. A computer-implemented method of inferring a property of a biomedical entity comprising:

   inputting two or more knowledge graphs related to biomedical entities in the form of vertices and edges and having different arities, at least one of the knowledge graphs being directional and at least one of the knowledge graphs being labelled;
   converting each of the knowledge graphs to a hyperedge representation as a list of unique edge identifiers, each with any source and target vertex vertices, any vertices connected by the edge in an undirected manner, and any labels associated with the edge;
   converting the hyperedge representations into a bipartite graph with the vertices and labels in one set and the edges in the other set, wherein the vertices and labels are linked to the edges as per the hyperedge representations by auxiliary edges A;
   sampling the bipartite graph to embed the bipartite graph as matrix entries in a feature matrix of the vertices, labels and edges; and
   using the feature matrix to infer a property of a biomedical entity in the combined knowledge graphs based on the matrix entries.

2. A method according to claim 1, wherein:
   the biomedical entity is a protein, and the knowledge graphs are related to proteins.

3. A method according to claim 1 or 2, wherein:
   one knowledge graph input is a directed labelled graph with binary-only relationships, such as protein interaction data and another knowledge graph input is a mixed directed/undirected labelled graph with ternary relationships such as a pathway database.

4. A method according to any of the preceding claims, wherein:
   sampling the bipartite graph includes:

   traversing the bipartite graph to provide possible sequences along the links between the sets;
   and embedding the bipartite graph includes:
   producing a feature matrix of the vertices, labels and edges from the sequences, with a co-occurrence score forming a matrix entry in each cell of the feature matrix; and wherein:
   the feature matrix is used to infer a property of a biomedical entity in the knowledge graph based on the co-occurrence scores.

5. A method according to any of the preceding claims, wherein:
   the hyperedge representations are combined before conversion into a bipartite graph, by considering vertices having the same name as the same vertex.

6. A method according to claim 5, wherein:
   a mapping tool is used to group alternative names for the same vertex together.

7. A method according to any of the preceding claims, wherein:
   each edge identifier has the form

$$(e,\ S,\ T,\ U)^l$$

   where $e$ is a unique edge identifier, $S$, $T$ are sets of source and target vertices, respectively, that are connected by the edge in a directed manner, $U$ is a set of vertices that are connected by the edge in an undirected manner, and $l$ is an edge label.

8. A method according to any of the preceding claims, wherein:
   an auxiliary edge A is formed between each vertex and an edge to which it is joined, with a relation between two vertices including two auxiliary edges, and the edge.

9. A method according to claim 8, wherein:

when an auxiliary edge is between a directed edge and a vertex, the auxiliary edge is directed in the same direction as the directed edge.

10. A method according to any of the preceding claims, wherein:
an auxiliary edge is formed between a label labelling an edge and the edge.

11. A method according to any of the preceding claims, wherein:
traversal of the bipartite graph produces sequences which alternate between a vertex or edge label and an edge of the hyperedge representations.

12. A method according to any of the preceding claims, wherein:
the feature matrix is populated by creating co-occurrence scores taking into account the distance in a sequence between two entities in the sequence and adding co-occurrence scores into the feature matrix for each sequence.

13. A method according to claim 12, further comprising:
using a context window starting at each point in a sequence but the last to give context sequences; wherein the co-occurrence scores between each starting point and the subsequent entities in the context sequence are calculated taking into account the distance between the entities in the context sequence and added into the feature matrix.

14. A method according to any of the preceding claims, wherein:
the property of the biomedical entity is inferred by calculation of a similarity of two or more biomedical entities, for example using cosine similarity.

15. A computer program which, when executed on a computer apparatus causes it to carry out the method according to any of the preceding claims.

16. An apparatus to infer a property of a protein comprising memory and a processor providing:
a bipartite converter, a graph sampler and a distributional analyser; wherein: the bipartite converter is to:

receive two or more knowledge graphs related to proteins in the form of vertices and edges and having different arities, at least one of the knowledge graphs being directional and at least one of the knowledge graphs being labelled;
convert each of the knowledge graphs to a hyperedge representation as a list of unique edge identifiers, each with any source and target vertex vertices, any vertices connected by the edge in an undirected manner, and any labels associated with the edge; and to
convert the hyperedge representations into a bipartite graph with the vertices and labels in one set and the edges in the other set, wherein the vertices and labels are linked to the edges as per the hyperedge representations by auxiliary edges $A$;
the graph sampler is to sample the bipartite graph; and
the distributional analyser is to:
embed the bipartite graph as matrix entries in a feature matrix of the vertices, labels and edges; to store the feature matrix in memory; and to use the feature matrix to infer a property of a biomedical entity in the combined knowledge graphs based on the matrix entries.

**Amended claims in accordance with Rule 137(2) EPC.**

1. A computer-implemented method of inferring a property of a biomedical entity comprising:

inputting two or more knowledge graphs related to biomedical entities in the form of vertices and edges and having different arities, at least one of the knowledge graphs being directional and at least one of the knowledge graphs being labelled;
converting each of the knowledge graphs to a hyperedge representation as a list of unique edge identifiers, each with any source and target vertex vertices, any vertices connected by the edge in an undirected manner, and any labels associated with the edge;
converting the hyperedge representations into a bipartite graph with the vertices and labels in one set and the edges in the other set, wherein the vertices and labels are linked to the edges as per the hyperedge representations by auxiliary edges A;

sampling the bipartite graph to embed the bipartite graph as matrix entries in a feature matrix of the vertices, labels and edges;
using the feature matrix to infer the property of the biomedical entity in the combined knowledge graphs based on the matrix entries; and
displaying a result of the inferring,
wherein:

the biomedical entity is a protein, and the knowledge graphs are related to proteins;
sampling the bipartite graph includes traversing the bipartite graph to provide possible sequences along the links between the sets;
embedding the bipartite graph includes producing the feature matrix of the vertices, labels and edges from the sequences, with a co-occurrence score forming a matrix entry in each cell of the feature matrix; and
the feature matrix is used to infer the property of the biomedical entity in the knowledge graph based on the co-occurrence scores.

**2.** A method according to claim 1, wherein:
one knowledge graph input is a directed labelled graph with binary-only relationships, such as protein interaction data and another knowledge graph input is a mixed directed/undirected labelled graph with ternary relationships such as a pathway database.

**3.** A method according to any of the preceding claims, wherein:
the hyperedge representations are combined before conversion into a bipartite graph, by considering vertices having the same name as the same vertex.

**4.** A method according to claim 3, wherein:
a mapping tool is used to group alternative names for the same vertex together.

**5.** A method according to any of the preceding claims, wherein:

each edge identifier has the form
$(e, S, T, U)^l$
where e is a unique edge identifier, $S, T$ are sets of source and target vertices, respectively, that are connected by the edge in a directed manner, $U$ is a set of vertices that are connected by the edge in an undirected manner, and $l$ is an edge label.

**6.** A method according to any of the preceding claims, wherein:
an auxiliary edge A is formed between each vertex and an edge to which it is joined, with a relation between two vertices including two auxiliary edges, and the edge.

**7.** A method according to claim 6, wherein:
when an auxiliary edge is between a directed edge and a vertex, the auxiliary edge is directed in the same direction as the directed edge.

**8.** A method according to any of the preceding claims, wherein:
an auxiliary edge is formed between a label labelling an edge and the edge.

**9.** A method according to any of the preceding claims, wherein:
traversal of the bipartite graph produces sequences which alternate between a vertex or edge label and an edge of the hyperedge representations.

**10.** A method according to any of the preceding claims, wherein:
the feature matrix is populated by creating co-occurrence scores taking into account the distance in a sequence between two entities in the sequence and adding co-occurrence scores into the feature matrix for each sequence.

**11.** A method according to claim 10, further comprising:
using a context window starting at each point in a sequence but the last to give context sequences; wherein the co-occurrence scores between each starting point and the subsequent entities in the context sequence are calculated taking into account the distance between the entities in the context sequence and added into the feature matrix.

**12.** A method according to any of the preceding claims, wherein:
the property of the biomedical entity is inferred by calculation of a similarity of two or more biomedical entities, for example using cosine similarity.

**13.** A computer program which, when executed on a computer apparatus causes it to carry out the method according to any of the preceding claims.

**14.** An apparatus to infer a property of a protein comprising memory (994) and a processor (993) providing:

a bipartite converter (10), a graph sampler (20), a distributional analyser (30) and
a graphical user interface, GUI (995, 996); wherein:

the bipartite converter (10) is to:

receive two or more knowledge graphs related to proteins in the form of vertices and edges and having different arities, at least one of the knowledge graphs being directional and at least one of the knowledge graphs being labelled;
convert each of the knowledge graphs to a hyperedge representation as a list of unique edge identifiers, each with any source and target vertex vertices, any vertices connected by the edge in an undirected manner, and any labels associated with the edge; and to
convert the hyperedge representations into a bipartite graph with the vertices and labels in one set and the edges in the other set, wherein the vertices and labels are linked to the edges as per the hyperedge representations by auxiliary edges A;
the graph sampler (20) is to sample the bipartite graph; and
the distributional analyser (30) is to:
embed the bipartite graph as matrix entries in a feature matrix of the vertices, labels and edges; to store the feature matrix in memory; and to use the feature matrix to infer the property of the protein in the combined knowledge graphs based on the matrix entries,
wherein:

the graph sampler (20) is to sampler the bipartite graph by traversing the bipartite graph to provide possible sequences along the links between the sets;
the distributional analyser (30) is to embed the bipartite graph by producing a feature matrix of the vertices, labels and edges from the sequences, with a co-occurrence score forming a matrix entry in each cell of the feature matrix;
the feature matrix is used to infer the property of the biomedical entity in the knowledge graph based on the co-occurrence scores; and
the GUI (995, 996) is to display a result of the inferring.

Start

S10

Input of one or more knowledge graphs

S12

Conversion of each knowledge graph
to hyperedge representations

S14

Conversion of the combined hyperedge
representations into a bipartite graph

S16

Bipartite graph traversal to provide
vertex and edge sequences

S18

Producing
a feature matrix from the sequences

S20

Infer protein or sequence property

End

Figure 1

Figure 2

FIG. 3

EP 3 640 864 A1

| # | Statements | Direct representation | Bipartite representation |
|---|---|---|---|
| 1 | $(e,\emptyset,\emptyset,\{a,b\})$<br>$(f,\emptyset,\emptyset,\{a,c\})$<br>$(g,\emptyset,\emptyset,\{b,c\})$<br>$(h,\emptyset,\emptyset,\{a,d\})$ | | |
| 2 | $(e,\{a\},\{b\},\emptyset)^r$<br>$(f,\{a\},\{c\},\emptyset)^r$<br>$(g,\{b\},\{c\},\emptyset)^s$<br>$(h,\{a\},\{d\},\emptyset)^s$ | | |
| 3 | $(e,\{a\},\{b\},\{D1\})^{r,0.5}$<br>$(f,\{a\},\{c\},\{D1\})^{r,1}$<br>$(g,\{b\},\{c\},\{D2\})^{s,0.7}$<br>$(h,\{a\},\{d\},\{D2\})^{s,0.1}$ | N/A | |

FIG. 4

Figure 5

Figure 6

Figure 7

Figure 8

Figure 9

EP 3 640 864 A1

(a)

(b)

$$\overline{V1} = \{u1, u2, u3, u4, u5, u6, u7, u8\}$$
$$\overline{E1} = \{$$
$$\quad (f15, \{u1\}, \{u5\}, \{\}),$$
$$\quad (f26, \{u2\}, \{u6\}, \{\}),$$
$$\quad (f37, \{u7\}, \{u3\}, \{\}),$$
$$\quad (f48, \{u4\}, \{u8\}, \{\})$$
$$\}$$

Figure 10

EP 3 640 864 A1

(a)

(b)

$\overline{V2}$ = {u0, u1, u2, u3, u4, c0, c1}
$\overline{E2}$ = {
   (f01, {u0}, {u1}, {c0}),
   (f02, {u0}, {u2}, {c0}),
   (f03, {u0}, {u3}, {c0}),
   (f04, {u0}, {u4}, {c1})
}

Figure 11

Start

S111

Input of one or more knowledge graphs

S112

Conversion of each knowledge graph
to hyperedge representations

S113

Combination hyperedge representations

S114

Conversion of the combined hyperedge
representations into a bipartite graph

End

Figure 12

(a)

(b)

$\overline{V}$ = {u0, u1, u2, u3, u4, u5, u6, u7, u8, c0, c1}
$\overline{E}$ = {
    (f01, {u0}, {u1}, {c0}),
    (f02, {u0}, {u2}, {c0}),
    (f03, {u0}, {u3}, {c0}),
    (f04, {u0}, {u4}, {c1}),
    (f15, {u1}, {u5}, {}),
    (f26, {u2}, {u6}, {}),
    (f37, {u7}, {u3}, {}),
    (f48, {u4}, {u8}, {})
}

Figure 13

40

EP 3 640 864 A1

(a)

(b)

V = {u0, u1, u2, u3, u4, u5, u6, u7, u8, c0, c1}

E = {f01, f02, f03, f04, f15, f26, f37, f48}

A = {

   (u0, →, f01), (u1, ←, f01), (c0, ↔, f01),

   (u0, →, f02), (u2, ←, f02), (c0, ↔, f02),

   (u0, →, f03), (u3, ←, f03), (c0, ↔, f03),

   (u0, →, f04), (u4, ←, f04), (c1, ↔, f04),

   (u1, →, f15), (u5, ←, f15),

   (u2, →, f26), (u6, ←, f26),

   (u3, ←, f37), (u7, →, f37),

   (u4, →, f48), (u8, ←, f48)

}

Figure 14

Figure 15

Figure 16

(a)

(b)

$\overline{V}$ = {u0, u1, u2, u3, u4, u5, u6, u7, u8}
$\overline{E}$ = {
   (f01, {u0}, {u1}, {}),
   (f02, {u0}, {u2}, {}),
   (f03, {u0}, {u3}, {}),
   (f04, {u0}, {u4}, {}),
   (f15, {u1}, {u5}, {}),
   (f26, {u2}, {u6}, {}),
   (f37, {u7}, {u3}, {}),
   (f48, {u4}, {u8}, {})
}

Figure 17

(a)

(b)

```
V = {u0, u1, u2, u3, u4, u5, u6, u7, u8}
E = {f01, f02, f03, f04, f15, f26, f37, f48}
A = {
    (u0, →, f01), (u1, ←, f01),
    (u0, →, f02), (u2, ←, f02),
    (u0, →, f03), (u3, ←, f03),
    (u0, →, f04), (u4, ←, f04),
    (u1, →, f15), (u5, ←, f15),
    (u2, →, f26), (u6, ←, f26),
    (u3, ←, f37), (u7, →, f37),
    (u4, →, f48), (u8, ←, f48)
}
```

Figure 18

Figure 19

$\overline{V}$ = {u0, u1, u2, u3, u4, u5, u6, u7, u8, c0, c1}
$\overline{E}$ = {
  (f01, {}, {}, {u0, u1, c0}),
  (f02, {}, {}, {u0, u2, c0}),
  (f03, {}, {}, {u0, u3, c0}),
  (f04, {}, {}, {u0, u4, c1}),
  (f15, {}, {}, {u1, u5}),
  (f26, {}, {}, {u2, u6}),
  (f37, {}, {}, {u7, u3}),
  (f48, {}, {}, {u4, u8})
}

Figure 20

EP 3 640 864 A1

(a)

(b)

```
V = {u0, u1, u2, u3, u4, u5, u6, u7, u8, c0, c1}
E = {f01, f02, f03, f04, f15, f26, f37, f48}
A = {
    (u0, ↔, f01), (u1, ↔, f01), (c0, ↔, f01),
    (u0, ↔, f02), (u2, ↔, f02), (c0, ↔, f02),
    (u0, ↔, f03), (u3, ↔, f03), (c0, ↔, f03),
    (u0, ↔, f04), (u4, ↔, f04), (c1, ↔, f04),
    (u1, ↔, f15), (u5, ↔, f15),
    (u2, ↔, f26), (u6, ↔, f26),
    (u3, ↔, f37), (u7, ↔, f37),
    (u4, ↔, f48), (u8, ↔, f48)
}
```

Figure 21

Figure 22

Figure 23

Figure 24

Start

S131

Input of sequences

S132

Initialisation of a co-occurrence matrix

S133

Having processed
all the sequences?

Yes

No

S134

Selection of an unprocessed sequence

S135

Update of the co-occurrence matrix

End

Figure 25

Figure 26

| | u0 | u1 | u2 | u3 | u4 | u5 | u6 | u7 | u8 | c0 | c1 | f01 | f02 | f03 | f04 | f15 | f26 | f37 | f48 |
|---|---|---|---|---|---|---|---|---|---|---|---|---|---|---|---|---|---|---|---|
| u0 | 0 | 1 | 0 | 0 | 0 | 1 | 0 | 0 | 0 | 0 | 0 | 1 | 0 | 0 | 0 | 1 | 0 | 0 | 0 |
| u1 | 1 | 0 | 0 | 0 | 0 | 1 | 0 | 0 | 0 | 0 | 0 | 1 | 0 | 0 | 0 | 1 | 0 | 0 | 0 |
| u2 | 0 | 0 | 0 | 0 | 0 | 0 | 0 | 0 | 0 | 0 | 0 | 0 | 0 | 0 | 0 | 0 | 0 | 0 | 0 |
| u3 | 0 | 0 | 0 | 0 | 0 | 0 | 0 | 0 | 0 | 0 | 0 | 0 | 0 | 0 | 0 | 0 | 0 | 0 | 0 |
| u4 | 0 | 0 | 0 | 0 | 0 | 0 | 0 | 0 | 0 | 0 | 0 | 0 | 0 | 0 | 0 | 0 | 0 | 0 | 0 |
| u5 | 1 | 1 | 0 | 0 | 0 | 0 | 0 | 0 | 0 | 0 | 0 | 1 | 0 | 0 | 0 | 1 | 0 | 0 | 0 |
| u6 | 0 | 0 | 0 | 0 | 0 | 0 | 0 | 0 | 0 | 0 | 0 | 0 | 0 | 0 | 0 | 0 | 0 | 0 | 0 |
| u7 | 0 | 0 | 0 | 0 | 0 | 0 | 0 | 0 | 0 | 0 | 0 | 0 | 0 | 0 | 0 | 0 | 0 | 0 | 0 |
| u8 | 0 | 0 | 0 | 0 | 0 | 0 | 0 | 0 | 0 | 0 | 0 | 0 | 0 | 0 | 0 | 0 | 0 | 0 | 0 |
| c0 | 0 | 0 | 0 | 0 | 0 | 0 | 0 | 0 | 0 | 0 | 0 | 0 | 0 | 0 | 0 | 0 | 0 | 0 | 0 |
| c1 | 0 | 0 | 0 | 0 | 0 | 0 | 0 | 0 | 0 | 0 | 0 | 0 | 0 | 0 | 0 | 0 | 0 | 0 | 0 |
| f01 | 1 | 1 | 0 | 0 | 0 | 1 | 0 | 0 | 0 | 0 | 0 | 0 | 0 | 0 | 0 | 1 | 0 | 0 | 0 |
| f02 | 0 | 0 | 0 | 0 | 0 | 0 | 0 | 0 | 0 | 0 | 0 | 0 | 0 | 0 | 0 | 0 | 0 | 0 | 0 |
| f03 | 0 | 0 | 0 | 0 | 0 | 0 | 0 | 0 | 0 | 0 | 0 | 0 | 0 | 0 | 0 | 0 | 0 | 0 | 0 |
| f04 | 0 | 0 | 0 | 0 | 0 | 0 | 0 | 0 | 0 | 0 | 0 | 0 | 0 | 0 | 0 | 0 | 0 | 0 | 0 |
| f15 | 1 | 1 | 0 | 0 | 0 | 1 | 0 | 0 | 0 | 0 | 0 | 1 | 0 | 0 | 0 | 0 | 0 | 0 | 0 |
| f26 | 0 | 0 | 0 | 0 | 0 | 0 | 0 | 0 | 0 | 0 | 0 | 0 | 0 | 0 | 0 | 0 | 0 | 0 | 0 |
| f37 | 0 | 0 | 0 | 0 | 0 | 0 | 0 | 0 | 0 | 0 | 0 | 0 | 0 | 0 | 0 | 0 | 0 | 0 | 0 |
| f48 | 0 | 0 | 0 | 0 | 0 | 0 | 0 | 0 | 0 | 0 | 0 | 0 | 0 | 0 | 0 | 0 | 0 | 0 | 0 |

Figure 27

|     | u0 | u1 | u2 | u3 | u4 | u5 | u6 | u7 | u8 | c0 | c1 | f01 | f02 | f03 | f04 | f15 | f26 | f37 | f48 |
|-----|----|----|----|----|----|----|----|----|----|----|----|-----|-----|-----|-----|-----|-----|-----|-----|
| u0  | 0  | 3  | 0  | 0  | 0  | 3  | 0  | 0  | 0  | 0  | 0  | 3   | 0   | 0   | 0   | 3   | 0   | 0   | 0   |
| u1  | 3  | 0  | 0  | 0  | 0  | 3  | 0  | 0  | 0  | 0  | 0  | 3   | 0   | 0   | 0   | 3   | 0   | 0   | 0   |
| u2  | 0  | 0  | 0  | 0  | 0  | 0  | 0  | 0  | 0  | 0  | 0  | 0   | 0   | 0   | 0   | 0   | 0   | 0   | 0   |
| u3  | 0  | 0  | 0  | 0  | 0  | 0  | 0  | 0  | 0  | 0  | 0  | 0   | 0   | 0   | 0   | 0   | 0   | 0   | 0   |
| u4  | 0  | 0  | 0  | 0  | 0  | 0  | 0  | 0  | 0  | 0  | 0  | 0   | 0   | 0   | 0   | 0   | 0   | 0   | 0   |
| u5  | 3  | 3  | 0  | 0  | 0  | 0  | 0  | 0  | 0  | 0  | 0  | 3   | 0   | 0   | 0   | 3   | 0   | 0   | 0   |
| u6  | 0  | 0  | 0  | 0  | 0  | 0  | 0  | 0  | 0  | 0  | 0  | 0   | 0   | 0   | 0   | 0   | 0   | 0   | 0   |
| u7  | 0  | 0  | 0  | 0  | 0  | 0  | 0  | 0  | 0  | 0  | 0  | 0   | 0   | 0   | 0   | 0   | 0   | 0   | 0   |
| u8  | 0  | 0  | 0  | 0  | 0  | 0  | 0  | 0  | 0  | 0  | 0  | 0   | 0   | 0   | 0   | 0   | 0   | 0   | 0   |
| c0  | 0  | 0  | 0  | 0  | 0  | 0  | 0  | 0  | 0  | 0  | 0  | 0   | 0   | 0   | 0   | 0   | 0   | 0   | 0   |
| c1  | 0  | 0  | 0  | 0  | 0  | 0  | 0  | 0  | 0  | 0  | 0  | 0   | 0   | 0   | 0   | 0   | 0   | 0   | 0   |
| f01 | 3  | 3  | 0  | 0  | 0  | 3  | 0  | 0  | 0  | 0  | 0  | 0   | 0   | 0   | 0   | 3   | 0   | 0   | 0   |
| f02 | 0  | 0  | 0  | 0  | 0  | 0  | 0  | 0  | 0  | 0  | 0  | 0   | 0   | 0   | 0   | 0   | 0   | 0   | 0   |
| f03 | 0  | 0  | 0  | 0  | 0  | 0  | 0  | 0  | 0  | 0  | 0  | 0   | 0   | 0   | 0   | 0   | 0   | 0   | 0   |
| f04 | 0  | 0  | 0  | 0  | 0  | 0  | 0  | 0  | 0  | 0  | 0  | 0   | 0   | 0   | 0   | 0   | 0   | 0   | 0   |
| f15 | 3  | 3  | 0  | 0  | 0  | 3  | 0  | 0  | 0  | 0  | 0  | 3   | 0   | 0   | 0   | 0   | 0   | 0   | 0   |
| f26 | 0  | 0  | 0  | 0  | 0  | 0  | 0  | 0  | 0  | 0  | 0  | 0   | 0   | 0   | 0   | 0   | 0   | 0   | 0   |
| f37 | 0  | 0  | 0  | 0  | 0  | 0  | 0  | 0  | 0  | 0  | 0  | 0   | 0   | 0   | 0   | 0   | 0   | 0   | 0   |
| f48 | 0  | 0  | 0  | 0  | 0  | 0  | 0  | 0  | 0  | 0  | 0  | 0   | 0   | 0   | 0   | 0   | 0   | 0   | 0   |

Figure 28

| | u0 | u1 | u2 | u3 | u4 | u5 | u6 | u7 | u8 | c0 | c1 | f01 | f02 | f03 | f04 | f15 | f26 | f37 | f48 |
|---|---|---|---|---|---|---|---|---|---|---|---|---|---|---|---|---|---|---|---|
| u0 | 0 | 4 | 0 | 0 | 0 | 3 | 0 | 0 | 0 | 1 | 0 | 5 | 0 | 0 | 0 | 3 | 0 | 0 | 0 |
| u1 | 4 | 0 | 0 | 0 | 0 | 3 | 0 | 0 | 0 | 1 | 0 | 5 | 0 | 0 | 0 | 3 | 0 | 0 | 0 |
| u2 | 0 | 0 | 0 | 0 | 0 | 0 | 0 | 0 | 0 | 0 | 0 | 0 | 0 | 0 | 0 | 0 | 0 | 0 | 0 |
| u3 | 0 | 0 | 0 | 0 | 0 | 0 | 0 | 0 | 0 | 0 | 0 | 0 | 0 | 0 | 0 | 0 | 0 | 0 | 0 |
| u4 | 0 | 0 | 0 | 0 | 0 | 0 | 0 | 0 | 0 | 0 | 0 | 0 | 0 | 0 | 0 | 0 | 0 | 0 | 0 |
| u5 | 3 | 3 | 0 | 0 | 0 | 0 | 0 | 0 | 0 | 0 | 0 | 3 | 0 | 0 | 0 | 3 | 0 | 0 | 0 |
| u6 | 0 | 0 | 0 | 0 | 0 | 0 | 0 | 0 | 0 | 0 | 0 | 0 | 0 | 0 | 0 | 0 | 0 | 0 | 0 |
| u7 | 0 | 0 | 0 | 0 | 0 | 0 | 0 | 0 | 0 | 0 | 0 | 0 | 0 | 0 | 0 | 0 | 0 | 0 | 0 |
| u8 | 0 | 0 | 0 | 0 | 0 | 0 | 0 | 0 | 0 | 0 | 0 | 0 | 0 | 0 | 0 | 0 | 0 | 0 | 0 |
| c0 | 1 | 1 | 0 | 0 | 0 | 0 | 0 | 0 | 0 | 0 | 0 | 2 | 0 | 0 | 0 | 0 | 0 | 0 | 0 |
| c1 | 0 | 0 | 0 | 0 | 0 | 0 | 0 | 0 | 0 | 0 | 0 | 0 | 0 | 0 | 0 | 0 | 0 | 0 | 0 |
| f01 | 5 | 5 | 0 | 0 | 0 | 3 | 0 | 0 | 0 | 2 | 0 | 0 | 0 | 0 | 0 | 3 | 0 | 0 | 0 |
| f02 | 0 | 0 | 0 | 0 | 0 | 0 | 0 | 0 | 0 | 0 | 0 | 0 | 0 | 0 | 0 | 0 | 0 | 0 | 0 |
| f03 | 0 | 0 | 0 | 0 | 0 | 0 | 0 | 0 | 0 | 0 | 0 | 0 | 0 | 0 | 0 | 0 | 0 | 0 | 0 |
| f04 | 0 | 0 | 0 | 0 | 0 | 0 | 0 | 0 | 0 | 0 | 0 | 0 | 0 | 0 | 0 | 0 | 0 | 0 | 0 |
| f15 | 3 | 3 | 0 | 0 | 0 | 3 | 0 | 0 | 0 | 0 | 0 | 3 | 0 | 0 | 0 | 0 | 0 | 0 | 0 |
| f26 | 0 | 0 | 0 | 0 | 0 | 0 | 0 | 0 | 0 | 0 | 0 | 0 | 0 | 0 | 0 | 0 | 0 | 0 | 0 |
| f37 | 0 | 0 | 0 | 0 | 0 | 0 | 0 | 0 | 0 | 0 | 0 | 0 | 0 | 0 | 0 | 0 | 0 | 0 | 0 |
| f48 | 0 | 0 | 0 | 0 | 0 | 0 | 0 | 0 | 0 | 0 | 0 | 0 | 0 | 0 | 0 | 0 | 0 | 0 | 0 |

Figure 29

|  | u0 | u1 | u2 | u3 | u4 | u5 | u6 | u7 | u8 | c0 | c1 | f01 | f02 | f03 | f04 | f15 | f26 | f37 | f48 |
|---|---|---|---|---|---|---|---|---|---|---|---|---|---|---|---|---|---|---|---|
| u0 | 0 | 6 | 6 | 6 | 6 | 3 | 3 | 0 | 3 | 9 | 3 | 9 | 9 | 9 | 9 | 3 | 3 | 0 | 3 |
| u1 | 6 | 0 | 0 | 0 | 0 | 35 | 0 | 0 | 0 | 11 | 0 | 15 | 1 | 1 | 0 | 35 | 0 | 0 | 0 |
| u2 | 6 | 0 | 0 | 0 | 0 | 0 | 35 | 0 | 0 | 11 | 0 | 1 | 15 | 1 | 0 | 0 | 35 | 0 | 0 |
| u3 | 6 | 0 | 0 | 0 | 0 | 0 | 0 | 24 | 0 | 11 | 0 | 1 | 1 | 15 | 0 | 0 | 0 | 24 | 0 |
| u4 | 6 | 0 | 0 | 0 | 0 | 0 | 0 | 0 | 51 | 0 | 27 | 0 | 0 | 0 | 33 | 0 | 0 | 0 | 51 |
| u5 | 3 | 35 | 0 | 0 | 0 | 0 | 0 | 0 | 0 | 8 | 0 | 11 | 0 | 0 | 0 | 35 | 0 | 0 | 0 |
| u6 | 3 | 0 | 35 | 0 | 0 | 0 | 0 | 0 | 0 | 8 | 0 | 0 | 11 | 0 | 0 | 0 | 35 | 0 | 0 |
| u7 | 0 | 0 | 0 | 24 | 0 | 0 | 0 | 0 | 0 | 0 | 0 | 0 | 0 | 0 | 0 | 0 | 0 | 24 | 0 |
| u8 | 3 | 0 | 0 | 0 | 51 | 0 | 0 | 0 | 0 | 0 | 24 | 0 | 0 | 0 | 27 | 0 | 0 | 0 | 51 |
| c0 | 9 | 11 | 11 | 11 | 0 | 8 | 8 | 0 | 0 | 0 | 0 | 14 | 14 | 14 | 0 | 8 | 8 | 0 | 0 |
| c1 | 3 | 0 | 0 | 0 | 27 | 0 | 0 | 0 | 24 | 0 | 0 | 0 | 0 | 0 | 30 | 0 | 0 | 0 | 24 |
| f01 | 9 | 15 | 1 | 1 | 0 | 11 | 0 | 0 | 0 | 14 | 0 | 0 | 2 | 2 | 0 | 11 | 0 | 0 | 0 |
| f02 | 9 | 1 | 15 | 1 | 0 | 0 | 11 | 0 | 0 | 14 | 0 | 2 | 0 | 2 | 0 | 0 | 11 | 0 | 0 |
| f03 | 9 | 1 | 1 | 15 | 0 | 0 | 0 | 0 | 0 | 14 | 0 | 2 | 2 | 0 | 0 | 0 | 0 | 0 | 0 |
| f04 | 9 | 0 | 0 | 0 | 33 | 0 | 0 | 0 | 27 | 0 | 30 | 0 | 0 | 0 | 0 | 0 | 0 | 0 | 27 |
| f15 | 3 | 35 | 0 | 0 | 0 | 35 | 0 | 0 | 0 | 8 | 0 | 11 | 0 | 0 | 0 | 0 | 0 | 0 | 0 |
| f26 | 3 | 0 | 35 | 0 | 0 | 0 | 35 | 0 | 0 | 8 | 0 | 0 | 11 | 0 | 0 | 0 | 0 | 0 | 0 |
| f37 | 0 | 0 | 0 | 24 | 0 | 0 | 0 | 24 | 0 | 0 | 0 | 0 | 0 | 0 | 0 | 0 | 0 | 0 | 0 |
| f48 | 3 | 0 | 0 | 0 | 51 | 0 | 0 | 0 | 51 | 0 | 24 | 0 | 0 | 0 | 27 | 0 | 0 | 0 | 0 |

Figure 30

| | u0 | u1 | u2 | u3 | u4 | u5 | u6 | u7 | u8 | c0 | c1 | f01 | f02 | f03 | f04 | f15 | f26 | f37 | f48 |
|---|---|---|---|---|---|---|---|---|---|---|---|---|---|---|---|---|---|---|---|
| u0 | 0 | 6 | 6 | 6 | 6 | 3 | 3 | 0 | 3 | 9 | 3 | 9 | 9 | 9 | 9 | 3 | 3 | 0 | 3 |
| u1 | 6 | 0 | 0 | 0 | 0 | 35 | 0 | 0 | 0 | 11 | 0 | 15 | 1 | 1 | 0 | 35 | 0 | 0 | 0 |
| u2 | 6 | 0 | 0 | 0 | 0 | 0 | 35 | 0 | 0 | 11 | 0 | 1 | 15 | 1 | 0 | 0 | 35 | 0 | 0 |
| u3 | 6 | 0 | 0 | 0 | 0 | 0 | 0 | 24 | 0 | 11 | 0 | 1 | 1 | 15 | 0 | 0 | 0 | 24 | 0 |
| u4 | 6 | 0 | 0 | 0 | 0 | 0 | 0 | 0 | 51 | 0 | 27 | 0 | 0 | 0 | 33 | 0 | 0 | 0 | 51 |
| u5 | 3 | 35 | 0 | 0 | 0 | 0 | 0 | 0 | 0 | 8 | 0 | 11 | 0 | 0 | 0 | 35 | 0 | 0 | 0 |
| u6 | 3 | 0 | 35 | 0 | 0 | 0 | 0 | 0 | 0 | 8 | 0 | 0 | 11 | 0 | 0 | 0 | 35 | 0 | 0 |
| u7 | 0 | 0 | 0 | 24 | 0 | 0 | 0 | 0 | 0 | 0 | 0 | 0 | 0 | 0 | 0 | 0 | 0 | 24 | 0 |
| u8 | 3 | 0 | 0 | 0 | 51 | 0 | 0 | 0 | 0 | 0 | 24 | 0 | 0 | 0 | 27 | 0 | 0 | 0 | 51 |
| c0 | 9 | 11 | 11 | 11 | 0 | 8 | 8 | 0 | 0 | 0 | 0 | 14 | 14 | 14 | 0 | 8 | 8 | 0 | 0 |
| c1 | 3 | 0 | 0 | 0 | 27 | 0 | 0 | 0 | 24 | 0 | 0 | 0 | 0 | 0 | 30 | 0 | 0 | 0 | 24 |
| f01 | 9 | 15 | 1 | 1 | 0 | 11 | 0 | 0 | 0 | 14 | 0 | 0 | 2 | 2 | 0 | 11 | 0 | 0 | 0 |
| f02 | 9 | 1 | 15 | 1 | 0 | 0 | 11 | 0 | 0 | 14 | 0 | 2 | 0 | 2 | 0 | 0 | 11 | 0 | 0 |
| f03 | 9 | 1 | 1 | 15 | 0 | 0 | 0 | 0 | 0 | 14 | 0 | 2 | 2 | 0 | 0 | 0 | 0 | 0 | 0 |
| f04 | 9 | 0 | 0 | 0 | 33 | 0 | 0 | 0 | 27 | 0 | 30 | 0 | 0 | 0 | 0 | 0 | 0 | 0 | 27 |
| f15 | 3 | 35 | 0 | 0 | 0 | 35 | 0 | 0 | 0 | 8 | 0 | 11 | 0 | 0 | 0 | 0 | 0 | 0 | 0 |
| f26 | 3 | 0 | 35 | 0 | 0 | 0 | 35 | 0 | 0 | 8 | 0 | 0 | 11 | 0 | 0 | 0 | 0 | 0 | 0 |
| f37 | 0 | 0 | 0 | 24 | 0 | 0 | 0 | 24 | 0 | 0 | 0 | 0 | 0 | 0 | 0 | 0 | 0 | 0 | 0 |
| f48 | 3 | 0 | 0 | 0 | 51 | 0 | 0 | 0 | 51 | 0 | 24 | 0 | 0 | 0 | 27 | 0 | 0 | 0 | 0 |

Figure 31

| | u0 | u1 | u2 | u3 | u4 | u5 | u6 | u7 | u8 | c0 | c1 | f01 | f02 | f03 | f04 | f15 | f26 | f37 | f48 |
|---|---|---|---|---|---|---|---|---|---|---|---|---|---|---|---|---|---|---|---|
| f48 | 3 | 0 | 0 | 0 | 51 | 0 | 0 | 0 | 51 | 0 | 24 | 0 | 0 | 0 | 27 | 0 | 0 | 0 | 0 |
| f37 | 0 | 0 | 0 | 24 | 0 | 0 | 0 | 24 | 0 | 0 | 0 | 0 | 0 | 0 | 0 | 0 | 0 | 0 | 0 |
| f26 | 3 | 0 | 35 | 0 | 0 | 0 | 35 | 0 | 0 | 8 | 0 | 0 | 11 | 0 | 0 | 0 | 0 | 0 | 0 |
| f15 | 3 | 35 | 0 | 0 | 0 | 35 | 0 | 0 | 0 | 8 | 0 | 11 | 0 | 0 | 0 | 0 | 0 | 0 | 0 |
| f04 | 9 | 0 | 0 | 0 | 33 | 0 | 0 | 0 | 27 | 0 | 30 | 0 | 0 | 0 | 0 | 0 | 0 | 0 | 27 |
| f03 | 9 | 1 | 1 | 15 | 0 | 0 | 0 | 0 | 0 | 14 | 0 | 2 | 2 | 0 | 0 | 0 | 0 | 0 | 0 |
| f02 | 9 | 1 | 15 | 1 | 0 | 0 | 11 | 0 | 0 | 14 | 0 | 2 | 0 | 2 | 0 | 0 | 11 | 0 | 0 |
| f01 | 9 | 15 | 1 | 1 | 0 | 11 | 0 | 0 | 0 | 14 | 0 | 0 | 2 | 2 | 0 | 11 | 0 | 0 | 0 |
| c1 | 3 | 0 | 0 | 0 | 27 | 0 | 0 | 0 | 24 | 0 | 0 | 0 | 0 | 0 | 30 | 0 | 0 | 0 | 24 |
| c0 | 9 | 11 | 11 | 11 | 0 | 8 | 8 | 0 | 0 | 0 | 0 | 14 | 14 | 14 | 0 | 8 | 8 | 0 | 0 |
| u8 | 3 | 0 | 0 | 0 | 51 | 0 | 0 | 0 | 0 | 0 | 24 | 0 | 0 | 0 | 27 | 0 | 0 | 0 | 51 |
| u7 | 0 | 0 | 0 | 24 | 0 | 0 | 0 | 0 | 0 | 0 | 0 | 0 | 0 | 0 | 0 | 0 | 0 | 24 | 0 |
| u6 | 3 | 0 | 35 | 0 | 0 | 0 | 0 | 0 | 0 | 8 | 0 | 0 | 11 | 0 | 0 | 0 | 35 | 0 | 0 |
| u5 | 3 | 35 | 0 | 0 | 0 | 0 | 0 | 0 | 0 | 8 | 0 | 11 | 0 | 0 | 0 | 35 | 0 | 0 | 0 |
| u4 | 6 | 0 | 0 | 0 | 0 | 0 | 0 | 0 | 51 | 0 | 27 | 0 | 0 | 0 | 33 | 0 | 0 | 0 | 51 |
| u3 | 6 | 0 | 0 | 0 | 0 | 0 | 0 | 24 | 0 | 11 | 0 | 1 | 1 | 15 | 0 | 0 | 0 | 24 | 0 |
| u2 | 6 | 0 | 0 | 0 | 0 | 0 | 35 | 0 | 0 | 11 | 0 | 1 | 15 | 1 | 0 | 0 | 35 | 0 | 0 |
| u1 | 6 | 0 | 0 | 0 | 0 | 35 | 0 | 0 | 0 | 11 | 0 | 15 | 1 | 1 | 0 | 35 | 0 | 0 | 0 |
| u0 | 0 | 6 | 6 | 6 | 6 | 3 | 3 | 0 | 3 | 9 | 3 | 9 | 9 | 9 | 9 | 3 | 3 | 0 | 3 |

Figure 32

Figure 33

Europäisches
Patentamt

European
Patent Office

Office européen
des brevets

# EUROPEAN SEARCH REPORT

Application Number

EP 18 20 1367

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 089 060 A1 (FUJITSU LTD [JP]) 2 November 2016 (2016-11-02) * abstract * * paragraph [0001] - paragraph [0034] * * paragraph [0041] - paragraph [0070]; figures 1-8 * | 1-16 | INV. G06N5/02 G06N5/00 G16B40/30 |
| X | KAROLINE FAUST ET AL: "Prediction of metabolic pathways from genome-scale metabolic networks", BIOSYSTEMS, NORTH-HOLLAND, AMSTERDAM, NL, vol. 105, no. 2, 5 May 2011 (2011-05-05), pages 109-121, XP028239766, ISSN: 0303-2647, DOI: 10.1016/J.BIOSYSTEMS.2011.05.004 [retrieved on 2011-05-27] * abstract * * section 1 - section 5; page 109 - page 114; figures 1-4 * * section 10.3; page 119 - page 120 * | 1-16 | |
| X | LIU HAISHAN ET AL: "Mining Biomedical Ontologies and Data Using RDF Hypergraphs", 2013 12TH INTERNATIONAL CONFERENCE ON MACHINE LEARNING AND APPLICATIONS, IEEE, vol. 1, 4 December 2013 (2013-12-04), pages 141-146, XP032586425, DOI: 10.1109/ICMLA.2013.31 [retrieved on 2014-04-08] * abstract * * section I. - section III.; page 141 - page 144; figures 1-3 * | 1-16 | TECHNICAL FIELDS SEARCHED (IPC) G06N G06F G16B |

-/--

The present search report has been drawn up for all claims

| Place of search | Date of completion of the search | Examiner |
|---|---|---|
| The Hague | 22 March 2019 | Hasnas, Sergiu |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WANG L ET AL: "Development of Hypergraph Theory", JOURNAL OF COMPUTER AND SYSTEMS SCIENCES INTERNATIONAL, PLEIADES PUBLISHING, MOSCOW, vol. 57, no. 1, 2 March 2018 (2018-03-02), pages 109-114, XP036444938, ISSN: 1064-2307, DOI: 10.1134/S1064230718010136 [retrieved on 2018-03-02] * abstract * * section 2.; page 110 - page 112 * | 1,7,15, 16 | |
| A | US 2017/228435 A1 (TACCHI RUGGERO ALTAIR [US] ET AL) 10 August 2017 (2017-08-10) * abstract * * paragraph [0043]; figure 1 * * paragraph [0082] * | 1,4, 12-16 | |
| | | | TECHNICAL FIELDS SEARCHED (IPC) |

The present search report has been drawn up for all claims

| Place of search | Date of completion of the search | Examiner |
|---|---|---|
| The Hague | 22 March 2019 | Hasnas, Sergiu |

EPO FORM 1503 03.82 (P04C01)

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

**EP 3 640 864 A1**

## ANNEX TO THE EUROPEAN SEARCH REPORT
## ON EUROPEAN PATENT APPLICATION NO.

EP 18 20 1367

This annex lists the patent family members relating to the patent documents cited in the above-mentioned European search report.
The members are as contained in the European Patent Office EDP file on
The European Patent Office is in no way liable for these particulars which are merely given for the purpose of information.

22-03-2019

| Patent document cited in search report | | Publication date | Patent family member(s) | | Publication date |
|---|---|---|---|---|---|
| EP 3089060 | A1 | 02-11-2016 | EP | 3089060 A1 | 02-11-2016 |
| | | | GB | 2537925 A | 02-11-2016 |
| | | | JP | 2016212853 A | 15-12-2016 |
| | | | US | 2016321407 A1 | 03-11-2016 |
| US 2017228435 | A1 | 10-08-2017 | US | 9436760 B1 | 06-09-2016 |
| | | | US | 2017228435 A1 | 10-08-2017 |
| | | | WO | 2017136687 A1 | 10-08-2017 |

EPO FORM P0459

For more details about this annex : see Official Journal of the European Patent Office, No. 12/82

**EP 3 640 864 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **JEFFREY PENNINGTON ; RICHARD SOCHER ; CHRISTOPHER D MANNING.** Glove: Global Vectors for Word Representation. *EMNLP,* 2014, vol. 14, 1532-43
- **TOMAS MIKOLOV et al.** Distributed representations of words and phrases and their compositionality. *Advances in neural information processing systems,* 2013, 3111-3119
- **QIONG YANG ; MO CHEN ; XIAOOU TANG.** Directed graph embedding. *Proceedings of IJCAI'07. IJCAI,* 2007
- Node2Vec: Scalable Feature Learning for Networks. **ADITYA GROVER ; JURE LESKOVEC.** Proceedings of the 22Nd ACM SIGKDD International Conference on Knowledge Discovery and Data Mining. ACM, 2016, 855-864
- RDF2vec: RDF graph embeddings for data mining. **PETAR RISTOSKI ; HEIKO PAULHEIM.** International Semantic Web Conference. Springer, 2016, 498-514
- On the Representation and Embedding of Knowledge Bases Beyond Binary Relations. **JIANFENG WEN et al.** Proceedings of IJCAI'16. IJCAI/AAAI Press, 2016, 1300-1307
- Distributed representations of words and phrases and their compositionality. **TOMAS MIKOLOV et al.** Advances in neural information processing systems. 2013, 3111-3119
- **ADRIAN BONDY ; UPPALURI SR MURTY.** Graph Theory (Graduate Texts in Mathematics 244). Springer, 2008
- Sampling from large graphs. **JURE LESKOVEC ; CHRISTOS FALOUTSOS.** Proceedings of the 12th ACM SIGKDD international conference on Knowledge discovery and data mining. ACM, 2006, 631-636
- **WEN et al.** *On the Representation and Embedding of Knowledge Bases Beyond Binary Relations,* 2013